(19) Europäisches Patentamt
European Patent Office
Office européen des brevets

(11) Veröffentlichungsnummer: **0 059 391**
**B1**

(12) # EUROPÄISCHE PATENTSCHRIFT

(45) Veröffentlichungstag der Patentschrift: **08.05.85**

(21) Anmeldenummer: **82101273.9**

(22) Anmeldetag: **19.02.82**

(51) Int. Cl.⁴: **C 07 D 487/14,** C 07 D 471/22, C 07 D 495/22, A 61 K 31/55 // (C07D487/14, 243:00, 235:00, 205:00), (C07D487/14, 243:00, 235:00, 209:00), (C07D471/22, 243:00, 235:00, 221:00, 205:00), (C07D471/22, 243:00, 235:00, 221:00, 209:00), (C07D495/22, 333:00)

(54) **Imidazodiazepine, Verfahren zu ihrer Herstellung und diese enthaltende Arzneimittel.**

(30) Priorität: **27.02.81 CH 1338/81**
**27.02.81 CH 1339/81**
**27.02.81 CH 1341/81**
**27.02.81 CH 1342/81**
**04.06.81 CH 3675/81**
**11.12.81 CH 7934/81**

(43) Veröffentlichungstag der Anmeldung: **08.09.82 Patentblatt 82/36**

(45) Bekanntmachung des Hinweises auf die Patenterteilung: **08.05.85 Patentblatt 85/19**

(84) Benannte Vertragsstaaten: **AT BE CH DE FR GB IT LI LU NL SE**

(56) Entgegenhaltungen:
**EP - A - 0 027 214**
**DE - A - 2 813 549**
**US - A - 3 933 794**

(73) Patentinhaber: **F. HOFFMANN-LA ROCHE & CO. Aktiengesellschaft, CH-4002 Basel (CH)**

(72) Erfinder: **Hunkeler, Walter, Dr., Im Stigler 32, CH-4465 Magden (CH)**
Erfinder: **Kyburz, Emilio, Dr., Unterer Rebbergweg 127, CH-4153 Reinach (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft Imidazodiazepine. Im speziellen betrifft sie tetracyclische Imidazodiazepine der allgemeinen Formel

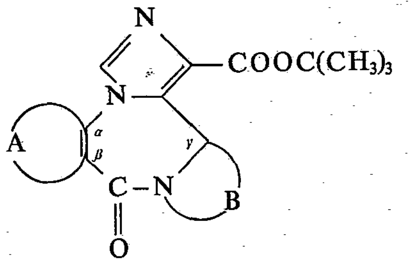

(I)

worin A zusammen mit den beiden mit $\alpha$ und $\beta$ bezeichneten Kohlenstoffatomen die Gruppe

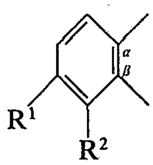 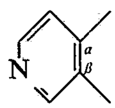 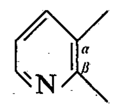 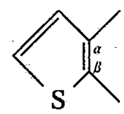

(a) (b) (c) oder (d)

B Dimethylen, Trimethylen oder Propenylen, $R^1$ Wasserstoff, Halogen, Trifluormethyl, Amino, Nitro, Cyano oder niederes Alkyl und $R^2$ Wasserstoff, Halogen, Trifluormethyl, Amino, Nitro, Cyano, niederes Alkyl, niederes Alkyloxy, niederes Alkylthio, niederes Alkylsulfinyl oder niederes Alkylsulfonyl bedeuten, und das mit $\gamma$ bezeichnete Kohlenstoffatom die (S)- oder (R, S)-Konfiguration aufweist, und pharmazeutisch annehmbare Säureadditionssalze davon.

Diese Verbindungen sind neu und zeichnen sich durch wertvolle pharmakodynamische Eigenschaften aus. Sie können bei der Bekämpfung bzw. Verhütung von Krankheiten verwendet werden.

Gegenstand der vorliegenden Erfindung sind Verbindungen der allgemeinen Formel I und pharmazeutisch annehmbare Säureadditionssalze davon als solche und als pharmazeutische Wirkstoffe, die Herstellung dieser Verbindungen, ferner Arzneimittel, enthaltend eine Verbindung der allgemeinen Formel I oder ein pharmazeutisch annehmbares Säureadditionssalz davon, und die Herstellung solcher Arzneimittel.

Die Ausdrücke »niederes Alkyl«, »niedere Alkylgruppe« und dergleichen bezeichnen gesättigte Kohlenwasserstoffreste, welche geradkettig oder verzweigt sein können, mit höchstens 7, vorzugsweise höchstens 4 Kohlenstoffatomen, wie Methyl, Äthyl, n-Propyl, Isopropyl, n-Butyl, s-Butyl, t-Butyl und dergleichen. Der Ausdruck »niederes Alkyloxy« bezeichnet über ein Sauerstoffatom gebundene niedere Alkylgruppen, wie z. B. Methoxy, Äthoxy, Isopropoxy und dergleichen. Der Ausdruck »niederes Alkylthio« bezeichnet über ein Schwefelatom gebundene niedere Alkylgruppen, wie z. B. Methylthio, Äthylthio, Isopropylthio und dergleichen. Der Ausdruck »niederes Alkylsulfinyl« bezeichnet über eine Sulfoxidgruppe gebundene niedere Alkylgruppen, wie z. B. Methylsulfinyl, Äthylsulfinyl, Isopropylsulfinyl und dergleichen. Der Ausdruck »niederes Alkylsulfonyl« bezeichnet über eine Sulfongruppe gebundene niedere Alkylgruppen, wie z. B. Methylsulfonyl, Äthylsulfonyl, Isopropylsulfonyl und dergleichen. Der Ausdruck »Halogen« bedeutet Fluor, Chlor, Brom oder Jod.

In einer speziellen Ausführungsform umfaßt die vorliegende Erfindung Verbindungen der obigen Formel I, worin A und B obige Bedeutung besitzen und $R^1$ Wasserstoff, Amino oder Halogen und $R^2$ Wasserstoff, Halogen, Trifluormethyl, niederes Alkyl, Cyano, Nitro, Amino, niederes Alkyloxy, niederes Alkylthio, niederes Alkylsulfinyl oder niederes Alkylsulfonyl bedeuten, und das mit $\gamma$ bezeichnete Kohlenstoffatom die (S)- oder (R,S)-Konfiguration aufweist.

Das Symbol A bedeutet vorzugsweise die Gruppe (a) wobei vorzugsweise $R^1$ Wasserstoff oder Halogen und $R^2$ Halogen, Trifluormethyl oder niederes Alkyl bedeuten. Das Symbol B bedeutet vorzugsweise Dimethylen oder Trimethylen. Das mit $\gamma$ bezeichnete Kohlenstoffatom weist vorzugsweise die (S)-Konfiguration auf.

Im Rahmen der vorliegenden Erfindung ganz besonders bevorzugte Verbindungen sind:

t-Butyl-(S)-12,12a-dihydro-9-oxo-8-(trifluormethyl)-9H,11H-azeto[2,1-c]imidazo-[1,5-a][1,4]benzodiazepin-1-carboxylat,

t-Butyl-(S)-12,12a-dihydro-8-methyl-9-oxo-9H,11H-azeto[2,1-c]imidazo[1,5-a][1,4]-benzodiazepin-1-carboxylat,

t-Butyl-(S)-8-chlor-7-fluor-11,12,13,13a-tetrahydro-9-oxo-9H-imidazo[1,5-a]pyrrolo-[2,1-c][1,4]benzodiazepin-1-carboxylat,

t-Butyl-(S)-8-chlor-11,12,13,13a-tetrahydro-9-oxo-9H-imidazo[1,5-a]pyrrolo[2,1-c][1,4]-benzodiazepin-1-carboxylat,
t-Butyl-(S)-8-äthyl-11,12,13,13a-tetrahydro-9-oxo-9H-imidazo[1,5-a]pyrrolo[2,1-c][1,4]-benzodiazepin-1-carboxylat,
t-Butyl-(S)-11,12,13,13a-tetrahydro-9-oxo-8-(trifluormethyl)-9H-imidazo[1,5-a]-pyrrolo[2,1-c][1,4]benzodiazepin-1-carboxylat,
t-Butyl-(S)-8-brom-11,12,13,13a-tetrahydro-9-oxo-9H-imidazo[1,5-a]pyrrolo[2,1-c][1,4]-benzodiazepin-1-carboxylat,
t-Butyl-(S)-11,12,13,13a-tetrahydro-8-jod-9-oxo-9H-imidazo[1,5-a]pyrrolo[2,1-c][1,4]-benzodiazepin-1-carboxylat,
t-Butyl-(S)-8-chlor-12,12a-dihydro-9-oxo-9H,11H-azeto[2,1-c]imidazo[1,5-a][1,4]-benzodiazepin-1-carboxylat und
t-Butyl-(S)-11,12,13,13a-tetrahydro-8-methyl-9-oxo-9H-imidazo[1,5-a]pyrrolo[2,1-c][1,4]-benzodiazepin-1-carboxylat.

Weitere bevorzugte, von der allgemeinen Formel I umfaßte Verbindungen sind:

t-Butyl-(S)-8-chlor-11,13a-dihydro-9-oxo-9H-imidazo[1,5-a]pyrrolo[2,1-c][1,4]-benzodiazepin-1-carboxylat,
t-Butyl-(S)-10,11,12,12a-tetrahydro-8-oxo-8H-imidazo[5,1-c]pyrrolo[1,2-a]thieno-[3,2-e][1,4]diazepin-1-carboxylat,
t-Butyl-(S)-11,12,13,13a-tetrahydro-9-oxo-9H-imidazo[1,5-a]pyrrolo[2,1-c][1,4]-benzodiazepin-1-carboxylat,
t-Butyl-(S)-7-chlor-11,12,13,13a-tetrahydro-9-oxo-9H-imidazo[1,5-a]pyrrolo[2,1-c][1,4]-benzodiazepin-1-carboxylat,
t-Butyl-(S)-8-amino-11,12,13,13a-tetrahydro-9-oxo-9H-imidazo[1,5-a]pyrrolo-[2,1-c][1,4]benzodiazepin-1-carboxylat,
t-Butyl-(S)-11,12,13,13a-tetrahydro-8-nitro-9-oxo-9H-imidazo[1,5-a]pyrrolo-[2,1-c][1,4]benzodiazepin-1-carboxylat,
t-Butyl-(S)-8-cyano-11,12,13,13a-tetrahydro-9-oxo-9H-imidazo[1,5-a]pyrrolo-[2,1-c][1,4]benzodiazepin-1-carboxylat,
t-Butyl-(S)-11,12,13,13a-tetrahydro-8-(methylthio)-9-oxo-9H-imidazo[1,5-a]-pyrrolo[2,1-c][1,4]benzodiazepin-1-carboxylat,
t-Butyl-(S)-11,12,13,13a-tetrahydro-8-(methylsulfonyl))-9-oxo-9H-imidazo[1,5-a]-pyrrolo[2,1-c][1,4]benzodiazepin-1-carboxylat,
t-Butyl-(S)-7-fluor-11,12,13,13a-tetrahydro-9-oxo-9H-imidazo[1,5-a]pyrrolo-[2,1-c][1,4]benzodiazepin-1-carboxylat und
t-Butyl-(S)-8-chlor-13,13a-dihydro-9-oxo-9H-imidazo[1,5-a]pyrrolo[2,1-c][1,4]-benzodiazepin-1-carboxylat.

Weitere repräsentative Vertreter der durch die allgemeine Formel I definierten Verbindungsklasse sind:

t-Butyl-(S)-8-brom-12,12a-dihydro-9-oxo-9H,11H-azeto-[2,1-c]imidazo[1,5-a][1,4]-benzodiazepin-1-carboxylat,
t-Butyl-(S)-12,12a-dihydro-8-jod-9-oxo-9H,11H-azeto[2,1-c]imidazo[1,5-a][1,4]-benzodiazepin-1-carboxylat,
t-Butyl-(S)-8-äthyl-12,12a-dihydro-9-oxo-9H,11H-azeto-[2,1-c]imidazo[1,5-a][1,4]-benzodiazepin-1-carboxylat,
t-Butyl-(S)-8-chlor-7-fluor-12,12a-dihydro-9-oxo-9H,11H-azeto[2,1-c]imidazo-[1,5-a][1,4]benzodiazepin-1-carboxylat,
t-Butyl-(S)-11,12,13,13a-tetrahydro-8-(methylsulfonyl)-9-oxo-9H-imidazo[1,5-a]-pyrrolo[2,1-c][1,4]benzodiazepin-1-carboxylat,
t-Butyl-(S)-11,12,13,13a-tetrahydro-8-methoxy-9-oxo-9H-imidazo[1,5-a]pyrrolo-[2,1-c][1,4]benzodiazepin-1-carboxylat,
t-Butyl-11,12,13,13a-tetrahydro-9-oxo-9H-imidazo[5,1-c]pyrido[3,2-e]pyrrolo-[1,2-a][1,4]diazepin-1-carboxylat,
t-Butyl-11,12,13,13a-tetrahydro-9-oxo-9H-imidazo[5,1-c]pyrido[4,3-e]pyrrolo-[1,2-a][1,4]diazepin-1-carboxylat,
t-Butyl-(S)-7-amino-11,12,13,13a-tetrahydro-9-oxo-9H-imidazo[1,5-a]pyrrolo[2,1-c][1,4]benzodiazepin-1-carboxylat und
t-Butyl-(S)-7-amino-8-brom-11,12,13,13a-tetrahydro-9-oxo-9H-imidazo[1,5-a]pyrrolo[2,1-c][1,4]benzodiazepin-1-carboxylat.

Die Imidazodiazepine der obigen Formel I und ihre pharmazeutisch annehmbaren Säureadditionssalze können erfindungsgemäß hergestellt werden, indem man

a) eine Verbindung der allgemeinen Formel

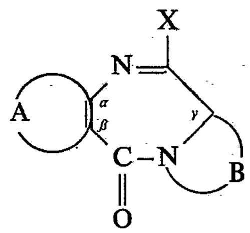

(II)

worin A und B obige Bedeutung besitzen, und X eine Abgangsgruppe bedeutet, in Gegenwart einer Base mit Isocyanessigsäure-t-butylester umsetzt, oder

b) eine Carbonsäure der allgemeinen Formel

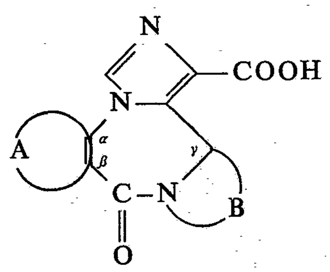

(III)

worin A und B obige Bedeutung besitzen, mit der Maßgabe, daß $R^1$ und/oder $R^2$ nicht Amino bedeuten, falls A die Gruppe (a) bedeutet, in den entsprechenden t-Butylester überführt, oder

c) in einer Verbindung der allgemeinen Formel

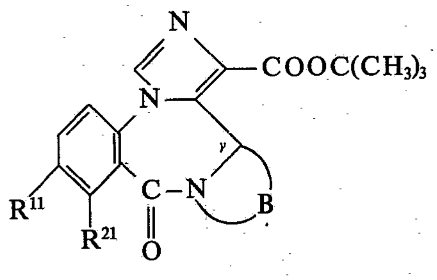

(Ia)

worin B obige Bedeutung besitzt, und entweder $R^{11}$ Halogen und $R^{21}$ Wasserstoff, Trifluormethyl, Amino, Nitro, Cyano oder niederes Alkyl, oder $R^{11}$ Wasserstoff, Trifluormethyl, Amino, Nitro, Cyano oder niederes Alkyl und $R^{21}$ Halogen bedeuten, das Halogenatom durch die Cyanogruppe oder, falls $R^{21}$ Halogen bedeutet, auch durch eine niedere Alkylthiogruppe ersetzt, oder

d) in einer Verbindung der allgemeinen Formel

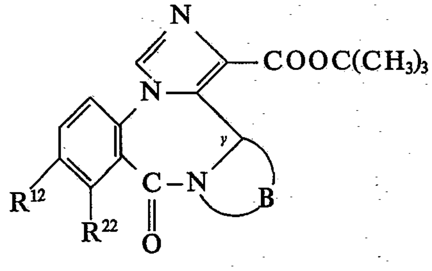

(Ib)

worin B obige Bedeutung besitzt und entweder $R^{12}$ Amino und $R^{22}$ Wasserstoff, Halogen, Trifluormethyl, Nitro, Cyano oder niederes Alkyl, oder $R^{12}$ Wasserstoff, Halogen, Trifluormethyl, Nitro, Cyano oder niederes Alkyl und $R^{22}$ Amino bedeuten, die Aminogruppe durch ein Wasserstoff- oder Halogenatom oder eine Cyano- oder Nitrogruppe ersetzt, oder

e) eine Verbindung der allgemeinen Formel

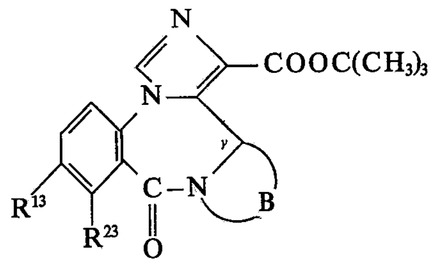

(Ic)

worin B obige Bedeutung besitzt und einer der Reste $R^{13}$ und $R^{23}$ Amino und der andere Wasserstoff bedeutet, in $\alpha$-Stellung zur Aminogruppe halogeniert, oder

f) in einer Verbindung der allgemeinen Formel

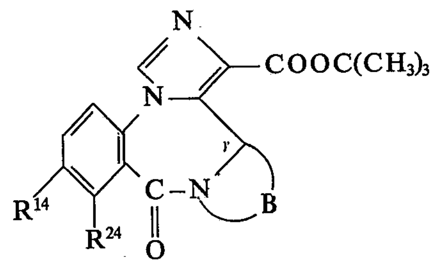

(Id)

worin $R^{14}$ Wasserstoff, Halogen, Trifluormethyl, Nitro, Cyano oder niederes Alkyl und $R^{24}$ niederes Alkylthio oder niederes Alkylsulfinyl bedeuten und B obige Bedeutung besitzt, die niedere Alkylthiogruppe zur niederen Alkylsulfonyl- oder Alkylsulfonylgruppe, bzw. die niedere Alkylsulfinylgruppe zur niederen Alkylsulfonylgruppe oxidiert, und

g) erwünschtenfalls eine erhaltene Verbindung der allgemeinen Formel I in ein pharmazeutisch annehmbares Säureadditionssalz überführt.

Gemäß Verfahrensvariante a) können Verbindungen der allgemeinen Formel I demnach aus Verbindungen der allgemeinen Formel II und Isocyanessigsäure-t-butylester hergestellt werden. Die in Formel II mit X bezeichnete Abgangsgruppe ist beispielsweise eine leicht abspaltbare Phosphinylgruppe, z. B. eine Gruppe der Formel

$$-O\overset{\overset{O}{\|}}{P}(OR^3)_2 \quad \text{oder} \quad -O\overset{\overset{O}{\|}}{P}(NR^4R^5)_2$$

worin $R^3$ niederes Alkyl und $R^4$ und $R^5$ je niederes Alkyl, Allyl, Phenyl oder substituiertes Phenyl oder zusammen mit dem Stickstoffatom einen unsubstituierten oder substituierten heterocyclischen Ring mit 3 bis 8 Gliedern (wie Morpholin) bedeuten,
ein Halogenatom, eine Alkylthiogruppe, eine Aralkylthiogruppe, eine N-Nitrosoalkylaminogruppe, eine Alkyloxygruppe, eine Mercaptogruppe und dergleichen (wenn X eine Mercaptogruppe bedeutet, so handelt es sich bei der entsprechenden Verbindung der Formel II um die Iminothiol-Form des entsprechenden Thiolactams). Die Reaktion einer Verbindung der allgemeinen Formel II mit Isocyanessigsäure-t-butylester erfolgt in einem inerten Lösungsmittel, wie Dimethylformamid, Hexamethylphosphorsäuretriamid, Dimethylsulfoxid, Tetrahydrofuran oder in irgend einem anderen geeigneten organischen Lösungsmittel und in Gegenwart einer Base, die genügend stark basisch ist, um das Anion des Isocyanessigsäure-t-butylesters zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmäßigerweise zwischen etwa $-40°$ C und etwa Raumtemperatur.

Gemäß Verfahrensvariante b) können Verbindungen der allgemeinen Formel I hergestellt werden, indem man Carbonsäuren der allgemeinen Formel III in die entsprechenden t-Butylester überführt. Diese Veresterung kann nach an sich bekannten und jedem Fachmann geläufigen Methoden durchgeführt werden; es gilt jedoch zu beachten, daß Verbindungen der Formel I, welche eine Aminogruppe als Strukturelement besitzen, nicht auf diesem Weg hergestellt werden können. Man kann beispielsweise eine Carbonsäure der Formel III mit einem geeigneten Reagens, z. B. mit Thionylchlorid, Phosphoroxychlorid, Oxalylchlorid oder dergleichen, in das entsprechende Carbonsäurechlorid überführen, und dieses in Gegenwart eines säurebindenden Mittels mit t-Butanol umsetzen. Als säurebindende Mittel eignen sich in erster Linie tertiäre Amine, wie Triäthylamin, Pyridin, Chinuclidin oder dergleichen. Unter Umständen kann die Anwesenheit einer katalytischen Menge an 4-Dimethylaminopyridin oder eines ähnlich reaktiven Amins vorteilhaft sein. Diese Veresterung kann in zwei getrennten Stufen, d. h. Bildung des reaktiven Carbonsäurederivates und Umsetzen desselben mit t-Butanol, oder in einem sogenannten Eintopfverfahren, was bevorzugt wird, durchgeführt werden. Als Lösungsmittel eignen sich z. B. halogenierte Kohlenwasserstoffe, wie Methylenchlorid, Chloroform, 1,2-Dichloräthan und dergleichen, Äther, wie Diäthyläther, t-Butylmethyläther, Tetrahydrofuran und dergleichen, Acetonitril, Dimethylformamid etc. Die Reaktionstemperatur liegt zweckmäßigerweise in einem Bereich von etwa −10° C bis Siedetemperatur des Reaktionsgemisches.

Es ist aber auch möglich, ein wie oben erhaltenes Carbonsäurechlorid oder ein Carbonsäureimidazolid, das durch Reaktion der freien Carbonsäure der Formel III mit N,N'-Carbonyldiimidazol leicht zugänglich ist, mit Natrium- oder Kalium-t-butoxid umzusetzen. Als Lösungsmittel eignen sich insbesondere Äther, wie Tetrahydrofuran und Dioxan, Dimethylformamid und dergleichen. Man arbeitet dabei, je nach verwendetem Lösungsmittel, in einem Bereich von etwa 0° bis etwa 100° C, vorzugsweise jedoch bei Raumtemperatur.

Gemäß Verfahrensvariante c) können Verbindungen der Formel I, worin A die Gruppe (a), und entweder $R^1$ Cyano und $R^2$ Wasserstoff, Trifluormethyl, Amino, Nitro, Cyano oder niederes Alkyl, oder $R^1$ Wasserstoff, Trifluormethyl, Amino, Nitro, Cyano oder niederes Alkyl und $R^2$ Cyano oder niederes Alkylthio bedeuten, hergestellt werden, indem man in Verbindungen der allgemeinen Formel Ia das Halogenatom durch die Cyanogruppe oder, falls $R^{21}$ Halogen bedeutet, auch durch eine niedere Alkylthiogruppe ersetzt. Dabei setzt man vorzugsweise eine entsprechende Brom- oder Jodverbindung der allgemeinen Formel Ia als Ausgangsstoff ein. Die Reaktion kann beispielsweise so durchgeführt werden, daß man die Verbindung der Formel Ia in einem inerten organischen Lösungsmittel mit Kupfer(I)cyanid oder einem niederen Alkylmercaptan in Gegenwart einer Base, welche genügend stark basisch ist, um aus dem Mercaptan das entsprechende Anion zu bilden, umsetzt. Geeignete Lösungsmittel sind beispielsweise Dimethylformamid und dergleichen. Die Reaktionstemperatur liegt zweckmäßigerweise in einem Bereich von etwa Raumtemperatur bis Siedetemperatur der Reaktionsmischung. Für die Herstellung des Anions aus einem niederen Alkylmercaptan geeignete Basen sind beispielsweise Natrium- oder Kaliumalkoholate, wie Natriumäthanolat und Kalium-t-butanolat, Natriumhydrid und dergleichen.

Gemäß Verfahrensvariante d) kann man in einer Verbindung der Formel Ib die Aminogruppe durch ein Wasserstoff- oder Halogenatom oder eine Cyano- oder Nitrogruppe ersetzen. Für den Ersatz durch ein Halogenatom oder eine Cyano- oder Nitrogruppe kann man so vorgehen, daß man die Aminoverbindung der Formel Ib in ein entsprechendes Diazoniumsalz überführt und dieses, gegebenenfalls ohne vorgängige Isolierung, mit einem Nitrit, wie Natriumnitrit, oder mit einem Halogenid, z. B. mit einem Chlorid oder Bromid, oder einem Cyanid in Gegenwart eines Kupfer(I)-salzes umsetzt. Für die Herstellung der entsprechenden Jodide ist die Anwesenheit eines Kupfer(I)salzes nicht erforderlich. Entsprechende Fluoride werden zweckmäßigerweise über die entsprechenden Diazonium-tetrafluoroborate hergestellt, beispielsweise durch Bestrahlen mit UV-Licht. Diese Reaktionen werden in wäßrigen Lösungen bei Temperaturen von etwa −10° C bis etwa Raumtemperatur durchgeführt.

Der Ersatz einer Aminogruppe in einer Verbindung der Formel Ib durch die Nitrogruppe kann aber auch dadurch bewerkstelligt werden, daß man eine Verbindung der Formel Ib oxidiert. Als Oxidationsmittel eignen sich beispielsweise Persäuren, wie Peressigsäure, Trifluorperessigsäure, m-Chlorperbenzoesäure und Perbenzoesäure, und dergleichen. Als Lösungsmittel kommen, je nach eingesetztem Oxidationsmittel, Carbonsäuren, wie Essigsäure etc., halogenierte Kohlenwasserstoffe, wie Methylenchlorid, Chloroform, 1,2-Dichloräthan etc., oder dergleichen in Frage. In der Regel arbeitet man bei Temperaturen von etwa 0° C bis etwa Raumtemperatur.

Der Ersatz der Aminogruppe durch ein Wasserstoffatom kann beispielsweise durch Reduktion eines entsprechenden Diazoniumsalzes bewerkstelligt werden, beispielsweise durch Erhitzen in einem cyclischen Äther, wie Tetrahydrofuran oder Dioxan, oder in Dimethylformamid; man arbeitet dabei vorzugsweise bei der Siedetemperatur der Reaktionsmischung. In einer besonders bevorzugten Ausführungsform setzt man jedoch ein Amin der Formel Ib in einem cyclischen Äther, wie Tetrahydrofuran oder Dioxan, mit t-Butylnitrit um, wobei man dabei vorzugsweise bei der Siedetemperatur des Reaktionsgemisches arbeitet.

Gemäß Verfahrensvariante e) kann man eine Verbindung der Formel Ic in $\alpha$-Stellung zur Aminogruppe halogenieren. Geeignete Halogenierungsmittel sind beispielsweise N-Chlorsuccinimid, N-Bromsuccinimid, N-Chloracetamid, N-Bromacetamid und dergleichen. Als Lösungsmittel verwendet man zweckmäßigerweise inerte organische Lösungsmittel, beispielsweise halogenierte Kohlenwasserstoffe, wie Methylenchlorid, 1,2-Dichloräthan, Chloroform und dergleichen, Dimethylformamid, Dimethylacetamid, Acetonitril, Äther, wie Diäthyläther, Tetrahydrofuran, Dioxan und dergleichen, usw. Die Halogenierung kann in einem Temperaturbereich von etwa 0° C bis Siedetemperatur der Reaktionsmischung durchgeführt werden, wobei ein Bereich von etwa Raumtemperatur bis etwa 100° C bevorzugt wird.

Gemäß Verfahrensvariante f) kann man in einer Verbindung der Formel Id die niedere Alkylthio- oder Alkylsulfinylgruppe oxidieren. Bei der Oxidation einer niederen Alkylthiogruppe erhält man je

nach verwendeten Reaktionsbedingungen, eine entsprechende Alkylsulfinyl- oder Alkylsulfonylverbindung. Geeignete Oxidationsmittel sind beispielsweise Persäuren, wie Peressigsäure, Trifluorperessigsäure, m-Chlorperbenzoesäure und Perbenzoesäure, Alkylhydroperoxide, wie t-Butylhydroperoxid, Wasserstoffperoxid und dergleichen. Als Lösungsmittel kommen, je nach eingesetztem Oxidationsmittel, Carbonsäuren, wie Essigsäure etc., halogenierte Kohlenwasserstoffe, wie Methylenchlorid, Chloroform, 1,2-Dichloräthan etc., aromatische Kohlenwasserstoffe, wie Benzol, Toluol und Xylol, oder dergleichen, in Frage. In der Regel arbeitet man bei Temperaturen von etwa 0° C bis Siedetemperatur des Reaktionsgemisches.

Durch die Einführung einer Alkylsulfinylgruppe erhält man ein zusätzliches optisch aktives Zentrum; die vorliegende Erfindung umfaßt alle möglichen Diastereoisomeren und Mischungen davon.

Gemäß Verfahrensvariante g) können Verbindungen der Formel I erfindungsgemäß in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der Formel II können ausgehend von Verbindungen der allgemeinen Formel

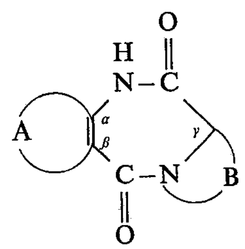

(IV)

worin A und B obige Bedeutung besitzen,
hergestellt werden, und zwar nach Methoden, welche an sich bekannt sind, vgl. z. B. die Belgischen Patentschriften No. 802 233, 833 249 und 865 653, die Amerikanische Patentschrift No. 3 681 341 und J. Org. Chemistry 29, 231 (1964).

Verschiedene der weiter unten folgenden Beispiele enthalten detaillierte Angaben betreffend die Herstellung von Verbindungen der Formel II aus Verbindungen der Formel IV.

Die Verbindungen der Formel IV ihrerseits sind bekannt oder nach an sich bekannten Methoden leicht herstellbar. Verbindungen der Formel IV, worin A die Gruppe (a) oder (d) bedeutet, können beispielsweise durch Umsetzen eines entsprechenden Carbonsäureanhydrids der allgemeinen Formel

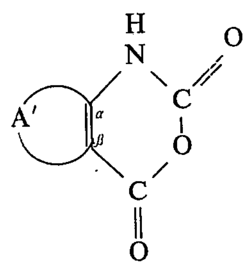

(V)

worin A′ die Gruppe (a) oder (d) bedeutet, mit der Maßgabe, daß $R^1$ und/oder $R^2$ nicht Amino bedeuten, falls A′ die Gruppe (a) bedeutet,
mit einer Aminosäure der allgemeinen Formel

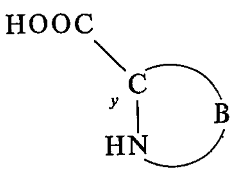

(VI)

worin B obige Bedeutung besitzt,
hergestellt werden.

Die Verbindungen der Formel IV, worin A die Gruppe (a) oder (d) bedeutet, können aber auch ausgehend von Verbindungen der allgemeinen Formel

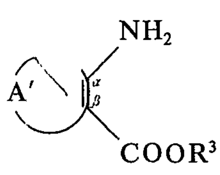

(VII)

worin $R^3$ niederes Alkyl bedeutet und A' obige Bedeutung besitzt, hergestellt werden, beispielsweise durch Umsetzen einer solchen Verbindung mit einem reaktiven Derivat einer Carbonsäure der allgemeinen Formel

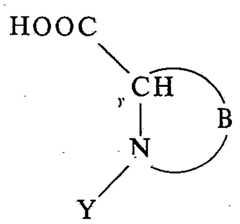

(VIII)

worin Y eine Schutzgruppe bedeutet und B obige Bedeutung besitzt, beispielsweise einem Carbonsäurechlorid oder dergleichen. Nach Entfernen der mit Y bezeichneten Schutzgruppe aus einer so erhaltenen Verbindung der allgemeinen Formel

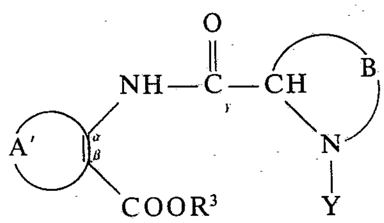

(IX)

worin A', B, $R^3$ und Y obige Bedeutung besitzen, und Cyclisieren, beispielsweise durch kurzzeitiges Erhitzen des erhaltenen Stoffes auf Temperaturen von etwa 100° bis etwa 300° C, erhält man die gewünschte Verbindung der Formel IV.

Verbindungen der Formel IV, worin A die Gruppe (b) oder (c) bedeutet, können hergestellt werden, indem man eine Verbindung der allgemeinen Formel

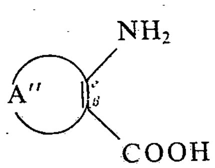

(X)

worin A'' die Gruppe (b) oder (c) bedeutet, in einem inerten organischen Lösungsmittel, wie Dimethylformamid, Tetrahydrofuran, Dioxan oder dergleichen, in Gegenwart von N,N'-Carbonyldiimidazol mit einer Verbindung der allgemeinen Formel

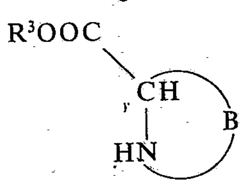

(XI)

worin B und $R^3$ obige Bedeutung besitzen, umsetzt. Diese Reaktion wird vorzugsweise als »Eintopfverfahren« durchgeführt, d. h. daß man das in einem ersten Schritt gebildete Carbonsäureimidazolid nicht isoliert, sondern direkt mit einer Verbindung der Formel XI umsetzt und den erhaltenen Stoff, beispielsweise durch kurzzeitiges Erhitzen auf Temperaturen von etwa 100° bis etwa 300° C, cyclisiert.

Verbindungen der allgemeinen Formel

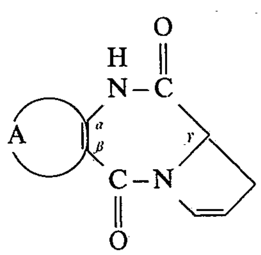

(IVa)

worin A obige Bedeutung besitzt, können auch hergestellt werden, indem man in an sich bekannter Weise in einer Verbindung der

allgemeinen Formel

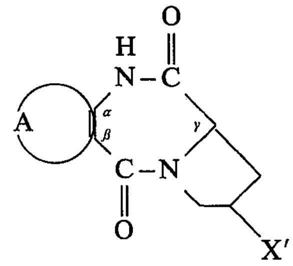

(XII)

worin A obige Bedeutung besitzt und X′ eine Abgangsgruppe bedeutet,
die mit X′ bezeichnete Abgangsgruppe eliminiert. Als Abgangsgruppe kommen beispielsweise Sulfonsäuregruppen, wie Methansulfonyloxy, p-Toluolsulfonyloxy und dergleichen, Halogenatome, wie Chlor, Brom und Jod, oder dergleichen in Frage. Die Abspaltung erfolgt mit einer Base, wie Natriumhydrid, in einem inerten organischen Lösungsmittel, wie Dimethylformamid.

Verbindungen der Formel XII können beispielsweise in Analogie zur Herstellung von Verbindungen der Formel IV aus Verbindungen der Formeln V und VI, bzw. aus solchen der Formeln X und XI hergestellt werden.

Verbindungen der Formel IV, worin A die Gruppe (a), einer der Reste $R^1$ und $R^2$ Halogen und der andere Wasserstoff, Trifluormethyl, Amino, Nitro, Cyano oder niederes Alkyl bedeuten, können durch Behandeln mit Kupfer(I)cyanid oder einem niederen Alkylmercaptan in Gegenwart einer Base in entsprechende Cyano- oder Alkylthioverbindungen übergeführt werden. Entsprechende niedere Alkylsulfinyl- und niedere Alkylsulfonylverbindungen können durch Oxidation entsprechender niederer Alkylthioverbindungen erhalten werden. Eine weitere Möglichkeit zur Abwandlung von Verbindungen der Formel IV, worin A die Gruppe (a) bedeutet, besteht darin, daß man eine solche Verbindung, worin einer der Reste $R^1$ und $R^2$ Amino und der andere Wasserstoff bedeutet, in $\alpha$-Stellung zur Aminogruppe halogeniert. Weiterhin kann man in einer Verbindung der Formel IV, worin A die Gruppe (a), einer der Reste $R^1$ und $R^2$ Amino und der andere Wasserstoff, Halogen, Trifluormethyl, Nitro, Cyano oder niederes Alkyl bedeuten, die Aminogruppe beispielsweise durch Reduktion eines entsprechenden Diazoniumsalzes abspalten, oder die Aminogruppe über ein entsprechendes Diazoniumsalz durch ein Halogenatom oder die Cyano- oder Nitrogruppe ersetzen, oder die Aminogruppe zur Nitrogruppe oxidieren. Schließlich kann man auch eine Verbindung der Formel IV, worin A die Gruppe (a) und $R^1$ und $R^2$ Wasserstoff bedeuten, nitrieren, wobei man eine entsprechende Verbindung der Formel IV erhält, worin $R^1$ Nitro und $R^2$ Wasserstoff bedeuten, oder eine entsprechende Verbindung, worin einer der Reste $R^1$ und $R^2$ Nitro bedeutet, zur entsprechenden Aminoverbindung reduzieren.

Die als Ausgangsstoffe verwendeten Verbindungen der Formel III können durch Hydrolyse der Estergruppe in Verbindungen der allgemeinen Formel

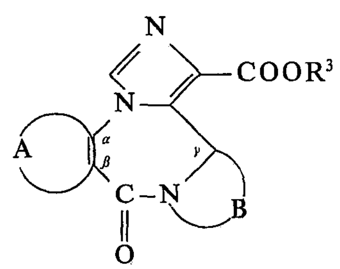

(XIII)

worin A, B und $R^3$ obige Bedeutung besitzen,
nach an sich bekannten und jedem Fachmann geläufigen Methoden leicht hergestellt werden.

Die Verbindungen der Formel XIII können hergestellt werden, indem man eine Verbindung der Formel II in Gegenwart einer Base mit einem Isocyanessigester der allgemeinen Formel

$$CN-CH_2-COOR^3 \qquad (XIV)$$

worin $R^3$ obige Bedeutung besitzt,
umsetzt, und zwar in Analogie zu Verfahrensvariante a).

Wie eingangs erwähnt sind die Verbindungen der Formel I neu und besitzen äußerst wertvolle pharmakodynamische Eigenschaften. Sie weisen nur eine geringe Toxizität auf, und es hat sich gezeigt, daß sie eine ausgeprägte antikonvulsive und anxiolytische Wirkung besitzen. Die anxiolytische Wirkung kann im nachstehend beschriebenen Tierversuch experimentell nachgewiesen werden.

Die Testapparatur ist eine Eintasten-Skinnerbox mit Futterpillengeber.

Während 3 einstündigen Vorversuchen (an drei verschiedenen Tagen) werden hungrige, 180—230 g schwere, weibliche Ratten darauf trainiert, die Taste des Futterpillengebers zu drücken, um Futterpillen von je 45 mg zu erhalten (jeder Tastendruck wird belohnt); im dritten Vorversuch erreichen die Ratten eine Rate von 150—200 Tastenbetätigungen pro Stunde.

In einem vierten Vorversuch wird jede durch Tastendruck hervorgerufene Pillenbelohnung mit einem kurzen elektrischen Fuß-Schock (1,0 mA) kombiniert. Die Ratten, welche mit dieser Konflikt-Situation konfrontiert sind, betätigen anfangs noch etwa 5—10mal die Drucktaste und hören dann aus Angst gänzlich auf.

In einem fünften Vorversuch können die Ratten die Taste des Futterpillengebers wieder ohne zusätzlichen Fuß-Schock drücken, wobei wiederum eine Rate von 150—200 Tastenbetätigungen pro Stunde erreicht wird.

In einem sechsten Vorversuch wird eine Selektion der Testtiere durchgeführt. Den Testtieren werden eine halbe Stunde vor Beginn dieses Vorversuchs peroral 10 mg/kg Chlordiazepoxid verabreicht; jede Pillenbelohnung wird wiederum mit einem Fuß-Schock kombiniert (Konflikt). Nur Ratten, die in diesem Vorversuch eine Rate von 20—50 Tastenbetätigungen erreichen (vgl. die 5—10 Tastenbetätigungen im vierten Vorversuch) werden als geeignete Testtiere für die Prüfung von potentiellen Anxioloytika behalten. Die Eliminationsrate in diesem Vorversuch beträgt 5%.

Im Hauptversuch werden zur Prüfung der potentiellen Anxiolytika pro Substanz und pro Dosis in der Regel 8 Ratten eingesetzt. Eine unbehandelte Kontrollgruppe ist nicht erforderlich, da jedes Tier als seine eigene Kontrolle dient.

Die Testsubstanzen, welche in einem Gemisch von 10 ml destilliertem Wasser und 2 Tropfen Tween 80 (Polyoxyäthylensorbitanmonooleat) gelöst oder suspendiert sind, werden den Tieren mit Hilfe einer Schlundsonde eine halbe Stunde vor dem einstündigen Hauptversuch verabreicht. Während des Hauptversuchs, in welchem bei jedem Tastendruck die Pillenbelohnung mit einem Fuß-Schock kombiniert ist (Konflikt), wird die Rate der Tastenbetätigungen pro Stunde registriert.

Die erste signifikant anxiolytisch wirksame Dosis wird mit dem Wilcoxon-Test (Vergleich von Paaren) bestimmt, indem die Anzahl der Tastenbetätigungen im Hauptversuch (Fuß-Schock, nach Vorbehandlung durch Testsubstanz) mit der Anzahl der Tastenbetätigungen im Kontrollversuch (Fuß-Schock, nach Vorbehandlung mit Kochsalzlösung) direkt verglichen wird.

Die nachfolgende Tabelle enthält für repräsentative Vertreter der durch die allgemeine Formel I definierten Verbindungsklasse die in obigem Versuch ermittelte erste signifikant anxiolytisch wirksame Dosis (ESD), sowie Angaben über deren akute Toxizität bei einmaliger oraler Verabreichung an Mäusen ($DL_{50}$ in mg/kg).

Tabelle

| Verbindung der Formel I, worin A | $R^1$ | $R^2$ | B | Konfiguration | ESD in mg/kg | $DL_{50}$ in mg/kg p.o. |
|---|---|---|---|---|---|---|
| (a) | H | Cl | $-(CH_2)_3-$ | (S) | 0,625 | 1250 |
| (a) | H | H | $-(CH_2)_3-$ | (S) | 10 | 2500 |
| (a) | H | Cl | $-(CH_2)_2-$ | (S) | 0,625 | 5000 |
| (a) | F | H | $-(CH_2)_3-$ | (S) | 5 | |
| (a) | H | $-SO_2CH_3$ | $-(CH_2)_3-$ | (S) | 5 | |
| (a) | H | $-SCH_3$ | $-(CH_2)_3$ | (S) | 1,25 | |
| (a) | H | $-CH_3$ | $-(CH_2)_3$ | (S) | 1,25 | 2500 |
| (d) | — | — | $-(CH_2)_3$ | (S) | 10 | |
| (a) | H | Cl | $-CH=CH-CH_2-$ | (R, S) | 10 | |
| (a) | H | $-CH_3$ | $-(CH_2)_2-$ | (S) | 2,5 | |
| (a) | H | J | $-(CH_2)_3-$ | (S) | <0,625 | >4000 |

Die Verbindungen der Formel I und ihre pharmazeutisch annehmbaren Säureadditionssalze können als Heilmittel, z. B. in Form pharmazeutischer Präparate, Verwendung finden. Die pharmazeutischen

Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von pharmazeutischen Präparaten können die erfindungsgemäßen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Öle, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Öle und dergleichen. Für Suppositorien eignen sich als Träger beispielsweise natürliche oder gehärtete Öle, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze, zur Veränderung des osmotischen Druckes, Puffer, Überzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, daß man eine oder mehrere Verbindungen der Formel I oder pharmazeutisch annehmbare Säureadditionssalze davon und gegebenenfalls einen oder mehrere andere therapeutisch wertvolle Stoffe in eine galenische Darreichungsform bringt. Wie eingangs erwähnt, können die Verbindungen der Formel I und ihre pharmazeutisch annehmbaren Säureadditionssalze bei der Bekämpfung bzw. Verhütung von Krankheiten verwendet werden und zwar insbesondere bei der Bekämpfung von Konvulsionen und Angstzuständen. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 0,1 mg bis 100 mg angemessen sein.

Die nachfolgenden Beispiele sollen die vorliegende Erfindung näher erläutern, ihren Umfang jedoch in keiner Weise beschränken. Sämtliche Temperaturen sind in Celsiusgrade angegeben.

## Beispiel 1

a) Man rührt 10 g (50,6 mMol) 6-Chlor-isatosäureanhydrid mit 5,82 g (50,6 mMol) L-Prolin in 80 ml Dimethylsulfoxid während 2 Stunden bei 110°. Die Lösung wird eingedampft und der Rückstand aus Äthanol auskristallisiert. Man erhält (S)-6-Chlor-1,2,3,11a-tetrahydro-5H-pyrrolo[2,1-c][1,4]-benzodiazepin-5,11(10H)-dion vom Schmelzpunkt 271—276°.

b) Man versetzt eine Suspension von 1,3 g (29,8 mMol) Natriumhydrid (55-proz. Öldispersion) in 40 ml trockenem Dimethylformamid bei 20 bis 30° unter Rühren mit 6,8 g (27,1 mMol) (S)-6-Chlor-1,2,3,11a-tetrahydro-5H-pyrrolo[2,1-c][1,4]-benzodiazepin-5,11(10H)-dion, rührt noch während 45 Minuten in obigem Temperaturbereich und tropft dann bei −35° 4,4 ml (27,1 mMol) Diäthylchlorphosphat dazu.

Inzwischen löst man 3,0 g (27,1 mMol) Kalium-t-butylat in 9,0 ml trockenem Dimethylformamid, kühlt in einem Aceton/Trockeneisbad ab, versetzt mit 3,9 g (27,1 mMol) Isocyanessigsäure-t-butylester und tropft die erhaltene Lösung bei −15° zum obigen Reaktionsgemisch. Man läßt auf 15° erwärmen, neutralisiert mit 1,5 ml Eisessig, gießt auf 100 ml Wasser und extrahiert viermal mit Methylenchlorid. Man wäscht die Methylenchloridlösung zweimal mit Wasser, trocknet über Magnesiumsulfat, dampft ein und chromatographiert das erhaltene Rohprodukt unter Eluieren mit Essigester an Kieselgel. Durch Umkristallisieren aus Essigester/n-Hexan erhält man t-Butyl-(S)-8-chlor-11,12,13,13a-tetrahydro-9-oxo-9H-imidazo[1,5-a]pyrrolo[2,1-c][1,4]benzodiazepin-1-carboxylat vom Schmelzpunkt 151—152° (eine zweite Modifikation schmilzt bei 216—217°).

## Beispiel 2

a) Man rührt ein Gemisch aus 4,8 g (24,3 mMol) 6-Chlorisatosäureanhydrid, 2,83 g (25 mMol) (L)-3,4-Dehydroprolin und 20 ml Dimethylsulfoxid während 1,25 Stunden bei 100°, gießt anschließend auf 200 ml Wasser und extrahiert dreimal mit Essigester. Die Essigesterlösung wird einmal mit Wasser gewaschen, über Magnesiumsulfat getrocknet, filtriert und eingedampft. Man chromatographiert den erhaltenen Rückstand an Kieselgel, kristallisiert anschließend aus Essigester und erhält (S)-6-Chlor-3,11a-dihydro-5H-pyrrolo-[2,1-c][1,4]benzodiazepin-5,11(10H)-dion vom

Schmelzpunkt 254—256°.

b) Man versetzt unter Rühren eine Suspension von 1,9 g (44,6 mMol) Natriumhydrid (55-proz. Öldispersion) in 80 ml trockenem Dimethylformamid bei —10° mit 10,0 g (40,2 mMol) (S)-6-Chlor-3,11a-dihydro-5H-pyrrolo-[2,1-c][1,4]benzodiazepin-5,11(10H)-dion, rührt während 1 Stunde und tropft anschließend bei —35° 7,7 ml (44,6 mMol) Diäthylchlorphosphat dazu.

Inzwischen löst man 4,9 g (44,4 mMol) Kalium-t-butylat in 15 ml trockenem Dimethylformamid, kühlt in einem Aceton/Trockeneisbad ab, versetzt mit 6,31 g (44,6 mMol) Isocyanessigsäure-t-butylester und gibt die erhaltene Lösung bei —15° tropfenweise zum obigen Reaktionsgemisch. Man läßt auf 15° erwärmen, neutralisiert mit 2,5 ml Eisessig, gießt auf 150 ml Wasser und extrahiert dreimal mit Methylenchlorid. Die organischen Auszüge werden zweimal mit Wasser gewaschen, über Magnesiumsulfat getrocknet und eingedampft. Das erhaltene Rohprodukt chromatographiert man unter Eluieren mit Essigester an Kieselgel. Nach Umkristallisieren aus Essigester/n-Hexan erhält man t-Butyl-8-chlor-11,13a-dihydro-9-oxo-9H-imidazo[1,5-a]pyrrolo[2,1-c][1,4]benzodiazepin-1-carboxylat vom Schmelzpunkt 227—229°.

## Beispiel 3

a) 11,3 g (0,057 Mol) 6-Chlorisatosäureanhydrid und 5,78 g (0,057 Mol) L-Azetidincarbonsäure werden in 50 ml Dimethylsulfoxid während 2 Stunden auf 125° erhitzt. Anschließend dampft man im Hochvakuum zur Trockene ein und erhitzt den erhaltenen Rückstand während 2 Stunden auf 150°. Durch Chromatographieren an Kieselgel unter Eluieren mit Essigester erhält man (S)-5-Chlor-1,10a-dihydroazeto[2,1-c][1,4]benzodiazepin-4,10(2H,9H)-dion vom Schmelzpunkt 225—228°.

b) Man versetzt eine Suspension von 0,47 g (10,8 mMol) Natriumhydrid (55-proz. Öldispersion) in 10 ml trockenem Dimethylformamid bei —15° und unter Rühren mit 2,12 g (9,0 mMol) (S)-5-Chlor-1,10a-dihydroazeto[2,1-c][1,4]benzodiazepin-4,10(2H,9H)-dion, rührt noch während 1 Stunde und tropft anschließend bei —35° 1,8 ml (10,8 mMol) Diäthylchlorphosphat dazu.

Inzwischen löst man 1,18 g (10,8 mMol) Kalium-t-butylat in 8 ml trockenem Dimethylformamid, kühlt in einem Aceton/Trockeneisbad ab, versetzt mit 1,52 g (10,8 mMol) Isocyanessigsäure-t-butylester und tropft die erhaltene Lösung bei —15° zum obigen Reaktionsgemisch. Man läßt auf 10° erwärmen, neutralisiert mit 0,6 ml Eisessig, gießt auf 80 ml Wasser und extrahiert dreimal mit Chloroform. Die Chloroformlösung wird zweimal mit Wasser gewaschen, über Magnesiumsulfat getrocknet und eingedampft. Das erhaltene Rohprodukt chromatographiert man unter Eluieren mit Essigester an Kieselgel, kristallisiert anschließend aus Essigester um und erhält t-Butyl-(S)-8-chlor-12,12a-dihydro-9-oxo-9H,11H-azeto[2,1-c]imidazo[1,5-a][1,4]benzodiazepin-1-carboxylat vom Schmelzpunkt 235—236°.

## Beispiel 4

a) Man erhitzt eine Mischung aus 175 g (0,93 Mol) 3-Amino-2-thiophencarbonsäuremethylester-hydrochlorid, 1,8 l n-Butanol und 77 g Natronlauge während 30 Minuten unter Rückfluß zum Sieden und engt die erhaltene Suspension ein. Das resultierende Gemisch aus Natriumsalz der 3-Amino-2-thiophencarbonsäure und Kochsalz versetzt man mit 800 ml Wasser, 280 ml konz. Salzsäure und 230 ml Tetrahydrofuran und leitet durch diese Mischung bei 15 bis 25° während 2,5 Stunden Phosgen und anschließend während 15 Minuten Luft ein. Der ausgefallene Festkörper wird abgenutscht, mit Wasser gewaschen und getrocknet. Man erhält 2H-Thieno[3,2-d][1,3]oxazin-2,4(1H)-dion vom Schmelzpunkt 220—221°.

b) Man rührt eine Lösung von 34,3 g (202 mMol) 2H-Thieno[3,2-d][1,3]oxazin-2,4(1H)-dion und 23,3 g (202 mMol) L-Prolin in 200 ml Dimethylsulfoxid während 1 Stunde bei 110°, gießt die erhaltene braune Lösung auf 2 l Wasser und rührt über Nacht bei Raumtemperatur. Das ausgefallene Produkt wird abgenutscht, im Vakuum getrocknet und mit ca. 200 ml siedendem Essigester gewaschen. Man erhält dabei (S)-5a,6,7,8-Tetrahydro-5H-pyrrolo-[1,2-a]thieno[3,2-e][1,4]diazepin-5,10(4H)-dion vom Schmelzpunkt 244—247°.

c) Man versetzt unter Rühren eine Suspension von 6,66 g (30 mMol) (S)-5a,6,7,8-Tetrahydro-5H-pyrrolo[1,2-a]thieno[3,2-e][1,4]diazepin-5,10(4H)-dion in 30 ml trockenem Dimethylformamid bei 0° mit 1,15 g (30 mMol) Natriumhydrid (55-proz. Öldispersion), rührt anschließend während 1 Stunde bei obiger Temperatur und tropft dann bei —30° 4,3 ml (30 mMol) Diäthylchlorphosphat dazu.

Inzwischen löst man 3,37 g (30 mMol) Kalium-t-butylat in 10 ml trockenem Dimethylformamid, kühlt in einem Aceton/Trockeneisbad ab, versetzt mit 4,23 g (30 mMol) Isocyanessigsäure-t-butylester und tropft die erhaltene Lösung bei —15° zum obigen Reaktionsgemisch. Man läßt auf 10° erwärmen, neutralisiert mit 3,3 ml Eisessig, gießt auf 300 ml Wasser und extrahiert dreimal mit Chloroform. Die Chloroformlösung wird viermal mit Wasser gewaschen, über Magnesiumsulfat getrocknet und eingedampft. Der zum Teil kristalline Rückstand wird zweimal aus Essigester umkristallisiert und liefert t-Butyl-(S)-10,11,12,12a-tetrahydro-8-oxo-8H-imidazo[5,1-c]pyrrolo[1,2-a]thieno[3,2-e][1,4]diazepin-1-carboxylat vom Schmelzpunkt 226—227°.

Beispiel 5

Man versetzt eine Suspension von 10,8 g (50,0 mMol) (S)-1,2,3,11a-Tetrahydro-5H-pyrrolo-[2,1-c][1,4]benzodiazepin-5,11(10H)-dion in 50 ml trockenem Dimethylformamid unter Rühren bei 0° mit 1,92 g (50 mMol) Natriumhydrid (55-proz. Öldispersion), rührt noch während 40 Minuten bei obiger Temperatur und tropft anschließend bei —25° 7,3 ml (50 mMol) Diäthylchlorphosphat dazu.

Inzwischen löst man 5,6 g (50 mMol) Kalium-t-butylat in 15 ml trockenem Dimethylformamid, kühlt in einem Aceton/Trockeneisbad ab, versetzt mit 7,0 g (50 mMol) Isocyanessigsäure-t-butylester und tropft die so erhaltene Lösung bei —15° zum obigen Reaktionsgemisch. Man läßt auf 10° erwärmen, neutralisiert mit 5,0 ml Eisessig, gießt auf 500 ml Wasser und rührt während 0,5 Stunden. Das ausgefallene Material wird abgenutscht, mit Wasser gewaschen, im Vakuum getrocknet und aus Essigester umkristallisiert. Man erhält t-Butyl-(S)-11,12,13,13a-tetrahydro-9-oxo-9H-imidazo[1,5-a]pyrrolo[2,1-c][1,4]benzodiazepin-1-carboxylat vom Schmelzpunkt 237—238°.

Beispiel 6

a) Eine Lösung von 13,6 g (0,0768 Mol) 6-Methylisatosäureanhydrid und 8,8 g (0,0768 Mol) L-Prolin in 75 ml Dimethylsulfoxid wird während 1 Stunde auf 110° erwärmt. Anschließend dampft man im Hochvakuum zur Trockene ein und kristallisiert den Rückstand unter Zusatz von Aktivkohle aus Essigester um. Man erhält (S)-1,2,3,11a-Tetrahydro-6-methyl-5H-pyrrolo-[2,1-c][1,4]benzodiazepin-5,11(10H)-dion vom Schmelzpunkt 212—214°.

b) Man versetzt unter Rühren eine Suspension von 2,51 g (57,5 mMol) Natriumhydrid (55-proz. Öldispersion) in 80 ml trockenem Dimethylformamid bei —20° mit 11,5 g (50 mMol) (S)-1,2,3,11a-Tetrahydro-6-methyl-5H-pyrrolo-[2,1-c][1,4]benzodiazepin-5,11(10H)-dion, rührt während 1 Stunde und versetzt die erhaltene Lösung bei —40° tropfenweise mit 8,6 ml (57,5 mMol) Diäthylchlorphosphat.

Inzwischen löst man 6,45 g (57,5 mMol) Kalium-t-butylat in 15 ml trockenem Dimethylformamid, kühlt in einem Aceton/Trockeneisbad ab und versetzt mit 8,12 g (57,5 mMol) Isocyanessigsäure-t-butylester und tropft die so erhaltene Lösung bei —15° zum obigen Reaktionsgemisch. Man läßt auf 10° erwärmen, neutralisiert mit 3,3 ml Eisessig, gießt auf 300 ml Wasser und extrahiert viermal mit Methylenchlorid. Die Methylenchloridlösung wird zweimal mit Wasser gewaschen, über Magnesiumsulfat getrocknet und eingedampft. Der Rückstand wird unter Eluieren mit Essigester, das 25% n-Hexan enthält, an Kieselgel chromatographiert. Nach Umkristallisieren aus Essigester erhält man t-Butyl-(S)-11,12,13,13a-tetrahydro-8-methyl-9-oxo-9H-imidazo[1,5-a]pyrrolo[2,1-c][1,4]benzodiazepin-1-carboxylat vom Schmelzpunkt 192—194°.

Beispiel 7

Man versetzt unter Rühren eine Suspension von 2,40 g (55 mMol) Natriumhydrid (55-proz. Öldispersion) in 80 ml trockenem Dimethylformamid bei —10° mit 12,0 g (47,9 mMol) (S)-7-Chlor-1,2,3,11a-tetrahydro-5H-pyrrolo[2,1-c][1,4]benzodiazepin-5,11(10H)-dion, rührt während 1 Stunde und versetzt die erhaltene Lösung bei —40° tropfenweise mit 9,2 ml (55 mMol) Diäthylchlorphosphat.

Inzwischen löst man 6,0 g (55 mMol) Kalium-t-butylat in 15 ml trockenem Dimethylformamid, kühlt in einem Aceton/Trockeneisbad ab, versetzt mit 7,74 g (55 mMol) Isocyanessigsäure-t-butylester und tropft die so erhaltene Lösung bei —15° zum obigen Reaktionsgemisch. Man entfernt das Kühlbad, rührt noch während 1 Stunde, neutralisiert anschließend mit 3,1 ml Eisessig, gießt auf 150 ml Wasser und extrahiert viermal mit Methylenchlorid. Man wäscht die organische Lösung zweimal mit Wasser, trocknet über Magnesiumsulfat und destilliert das Lösungsmittel ab. Man chromatographiert den Rückstand unter Eluieren mit Methylenchlorid, das 5% Essigester enthält, an Kieselgel und kristallisiert aus Essigester um. Man erhält t-Butyl-(S)-7-chlor-11,12,13,13a-tetrahydro-9-oxo-9H-imidazo[1,5-a]pyrrolo[2,1-c][1,4]benzodiazepin-1-carboxylat vom Schmelzpunkt 157—158°.

Beispiel 8

a) Man erhitzt 9,2 g (0,038 Mol) 6-Brom-isatosäureanhydrid und 4,6 g (0,040 Mol) L-Prolin in 55 ml Dimethylsulfoxid während 1 Stunde auf 70°, entfernt das Lösungsmittel im Hochvakuum und

erhitzt das erhaltene Öl während 15 Minuten auf 170°. Das Rohprodukt wird durch Chromatographieren an Kieselgel gereinigt, wobei man als Elutionsmittel ein Gemisch aus Chloroform und Methanol (20 : 1) verwendet. Man erhält (S)-6-Brom-1,2,3,11a-tetrahydro-5H-pyrrolo-[2,1-c][1,4]benzodiazepin-5,11(10H)-dion, das nach Umkristallisieren aus Chloroform/Hexan bei 221—224° schmilzt.

b) Man versetzt eine Lösung von 9,94 g (33,7 mMol) (S)-6-Brom-1,2,3,11a-tetrahydro-5H-pyrrolo-[2,1-c][1,4]benzodiazepin-5,11(10H)-dion in 30 ml trockenem Dimethylformamid bei —20 bis —10° unter Rühren mit 1,62 g (37 mMol) Natriumhydrid (55-proz. Öldispersion), rührt noch während 1,25 Stunden in obigem Temperaturbereich und tropft dann bei —40° 5,5 ml (37 mMol) Diäthylchlorphosphat dazu.

Inzwischen löst man 4,15 g (37 mMol) Kalium-t-butylat in 10 ml trockenem Dimethylformamid, kühlt in einem Aceton/Trockeneisbad ab, versetzt mit 5,22 g (37 mMol) Isocyanessigsäure-t-butylester und gibt die erhaltene Lösung bei — 15° tropfenweise zum obigen Reaktionsgemisch. Man läßt auf 10° erwärmen, neutralisiert mit 2,1 ml Eisessig, gießt auf 150 ml Wasser und extrahiert dreimal mit Methylenchlorid. Die Methylenchloridlösung wird zweimal mit Wasser gewaschen, über Magnesiumsulfat getrocknet und eingedampft. Das erhaltene Rohprodukt chromatographiert man unter Eluieren mit Essigester an Kieselgel. Durch Umkristallisieren aus Essigester erhält man t-Butyl-(S)-8-brom-11,12,13,13a-tetrahydro-9-oxo-9H-imidazo[1,5-a]pyrrolo[2,1-c][1,4]benzodiazepin-1-carboxylat vom Schmelzpunkt 206—208°.

Beispiel 9

a) Man erwärmt 14,6 g (0,050 Mol) 6-Jod-isatosäureanhydrid und 6,6 g (0,058 Mol) L-Prolin in 50 ml Dimethylsulfoxid während 30 Minuten auf 70°, entfernt das Lösungsmittel im Hochvakuum und erhitzt das erhaltene Öl während 15 Minuten auf 170°. Das Rohprodukt wird durch Chromatographieren an Kieselgel gereingt, wobei man als Elutionsmittel Methylenchlorid und ein Gemisch aus Methylenchlorid und Essigester verwendet. Man erhält (S)-1,2,3,11a-Tetrahydro-6-jod-5H-pyrrolo[2,1-c][1,4]benzodiazepin-5,11(10H)-dion, das nach Umkristallisieren aus Methanol bei 212—215° schmilzt.

b) Man versetzt eine Lösung von 10,0 g (29,2 mMol) (S)-1,2,3,11a-Tetrahydro-6-jod-5H-pyrrolo-[2,1-c][1,4]benzodiazepin-5,11(10H)-dion in 30 ml trockenem Dimethylformamid bei —20° unter Rühren mit 1,4 g (32,1 mMol) Natriumhydrid (55-proz. Öldispersion), rührt noch während 1 Stunde bei obiger Temperatur und tropft anschließend bei —45° 4,8 ml (32,1 mMol) Diäthylchlorphosphat dazu.

Inzwischen löst man 3,6 g (32,1 mMol) Kalium-t-butylat in 8 ml trockenem Dimethylformamid, kühlt in einem Aceton/Trockeneisbad ab, versetzt mit 4,5 g (32,1 mMol) Isocyanessigsäure-t-butylester und tropft die erhaltene Lösung bei —20° zum obigen Reaktionsgemisch. Man rührt noch während 15 Minuten bei —20°, neutralisiert mit 1,9 ml Eisessig, gießt auf 150 ml Wasser und extrahiert dreimal mit Methylenchlorid. Die organischen Auszüge werden zweimal mit Wasser gewaschen, über Magnesiumsulfat getrocknet und eingedampft. Das erhaltene Rohprodukt chromatographiert man unter Eluieren mit Essigester, das 30% n-Hexan enthält, an Kieselgel und kristallisiert anschließend aus Essigester um. Man erhält t-Butyl-(S)-11,12,13,13a-tetrahydro-8-jod-9-oxo-9H-imidazo[1,5-a]pyrrolo[2,1-c][1,4]benzodiazepin-1-carboxylat vom Schmelzpunkt 241—242°.

Beispiel 10

a) Man erhitzt 10,6 g (50,9 mMol) 6-Nitro-isatosäureanhydrid und 6,1 g (50,9 mMol) L-Prolin in 70 ml Dimethylsulfoxid während 45 Minuten auf 90°, dampft anschließend im Hochvakuum ein und erhitzt den erhaltenen Rückstand während 4 Stunden auf 140°. Man nimmt das kristalline Rohprodukt in 100 ml siedendem Äthanol auf, läßt über Nacht in der Kälte stehen, nutscht das erhaltene Material unter Nachwaschen mit kaltem Äthanol ab und trocknet es bis zur Gewichtskonstanz. Man erhält (S)-1,2,3,11a-Tetrahydro-6-nitro-5H-pyrrolo[2,1-c][1,4]benzodiazepin-5,11(10H)-dion mit einem Zersetzungspunkt von 235—237°.

b) 57,3 g (219,3 mMol) (S)-1,2,3,11a-Tetrahydro-6-nitro-5H-pyrrolo[2,1-c][1,4]benzodiazepin-5,11(10H)-dion werden in 1,2 l Methanol mit 3 g 10-proz. Palladiumkohle bei Raumtemperatur und Normaldruck hydriert. Nach beendeter Wasserstoffaufnahme wird zum Sieden erwärmt und vom Katalysator abgenutscht, wonach man das Filtrat eindampft. Durch Umkristallisieren aus Methanol erhält man (S)-6-Amino-1,2,3,11a-tetrahydro-5H-pyrrolo[2,1-c][1,4]benzodiazepin-5,11(10H)-dion vom Schmelzpunkt 246—248°.

c) Man versetzt eine Suspension von 26,8 g (115,9 mMol) (S)-6-Amino-1,2,3,11a-tetrahydro-5H-pyrrolo[2,1-c][1,4]benzodiazepin-5,11(10H)-dion in 80 ml trockenem Dimethylformamid bei —20 bis

−10° unter Rühren mit 5,56 g (127,4 mMol) Natriumhydrid (55-proz. Öldispersion), rührt noch während 1 Stunde in obigem Temperaturbereich und tropft dann bei −45° 19 ml (127,4 mMol) Diäthylchlorphosphat dazu.

Inzwischen löst man 16,5 g (127,4 mMol) Kalium-t-butylat in 23 ml trockenem Dimethylformamid, kühlt in einem Aceton/Trockeneisbad ab, versetzt mit 18 g (127,4 mMol) Isocyanessigsäure-t-butylester und gibt die erhaltene Lösung bei −20° tropfenweise zum obigen Reaktionsgemisch. Man läßt auf 5° erwärmen, neutralisiert mit 7,3 ml Eisessig, gießt auf 500 ml Wasser und extrahiert dreimal mit Methylenchlorid. Man wäscht die Methylenchloridlösung einmal mit Wasser und einmal mit gesättigter Natriumchloridlösung, trocknet über Magnesiumsulfat und dampft ein. Das Rohprodukt wird aus Essigester/Diäthyläther kristallisiert. Durch Umkristallisieren aus Essigester/n-Hexan erhält man t-Butyl-(S)-8-amino-11,12,13,13a-tetrahydro-9-oxo-9H-imidazo[1,5-a]pyrrolo[2,1-c][1,4]benzodiazepin-1-carboxylat mit einem Zersetzungspunkt von 223—224°.

## Beispiel 11

Man löst 4,06 g (21,1 mMol) ca. 90-proz. m-Chlorperbenzosäure in 50 ml Methylenchlorid, kühlt auf 0° ab, versetzt die erhaltene Suspension portionenweise mit 2,5 g (7,1 mMol) t-Butyl-(S)-8-amino-11,12,13,13a-tetrahydro-9-oxo-9H-imidazo[1,5-a]pyrrolo[2,1-c][1,4]benzodiazepin-1-carboxylat und rührt noch während 1 Stunde ohne Kühlung. Man gießt anschließend auf ca. 70 ml Eiswasser, stellt mit gesättigter Natriumbicarbonatlösung alkalisch, wäscht die Methylenchloridlösung dreimal mit gesättigter Natriumbicarbonatlösung und zweimal mit Wasser, trocknet über Magnesiumsulfat und dampft ein. Den erhaltenen Rückstand chromatographiert man unter Eluieren mit Essigester an Kieselgel. Durch Umkristallisieren aus Essigester erhält man t-Butyl-(S)-11,12,13,13a-tetrahydro-8-nitro-9-oxo-9H-imidazo[1,5-a]pyrrolo[2,1-c][1,4]benzodiazepin-1-carboxylat vom Schmelzpunkt 231—233°.

## Beispiel 12

Man löst 1,4 g (5,6 mMol) Kupfersulfat.5 $H_2O$ bei 50—60° in 5 ml Wasser, tropft nacheinander eine Lösung von 0,353 g (2,8 mMol) wasserfreiem Natriumsulfit in 2 ml Wasser und 0,411 g (8,4 mMol) Natriumcyanid in 1,5 ml Wasser dazu und rührt noch während 10 Minuten bei obiger Temperatur. Anschließend kühlt man in einem Eisbad ab, nutscht den entstandenen Niederschlag unter Nachwaschen mit Wasser ab und nimmt ihn in einer Lösung von 0,74 g (15,1 mMol) Natriumcyanid in 3,5 ml Wasser auf.

Inzwischen löst man 1,5 g (4,2 mMol) t-Butyl-(S)-8-amino-11,12,13,13a-tetrahydro-9-oxo-9H-imidazo[1,5-a]pyrrolo[2,1-c][1,4]benzodiazepin-1-carboxylat in einem Gemisch aus 1,04 ml konzentrierter Salzsäure und 2,1 ml Wasser auf und tropft bei 0—5° eine Lösung von 0,3 g (4,3 mMol) Natriumnitrit in 1,7 ml Wasser dazu. Die erhaltene Diazoniumsalzlösung tropft man bei 0° zur obigen Kupfer(I)cyanidlösung, erwärmt dann langsam auf 70° und rührt noch während 1 Stunde bei dieser Temperatur. Anschließend kühlt man in einem Eisbad ab und extrahiert das Reaktionsgemisch zweimal mit Essigester. Die organischen Auszüge wäscht man nacheinander einmal mit 2N-Natronlauge, einmal mit Wasser und einmal mit gesättiger Natriumchloridlösung, trocknet über Magnesiumsulfat und dampft ein. Der erhaltene Rückstand wird unter Eluieren mit Chloroform, das 1,5% Methanol enthält, an Kieselgel chromatographiert. Nach Umkristallisieren aus Essigester erhält man t-Butyl-(S)-8-cyano-11,12,13,13a-tetrahydro-9-oxo-9H-imdazo[1,5-a]pyrrolo[2,1-c][1,4]benzodiazepin-1-carboxylat mit einem Zersetzungspunkt von 249°.

## Beispiel 13

a) Man erhitzt 11,55 g (0,05 Mol) 6-(Trifluormethyl)-isatosäureanhydrid und 5,75 g (0,05 Mol) L-Prolin in 100 ml Dimethylsulfoxid während 1 Stunde auf 70°, entfernt das Lösungsmittel im Hochvakuum und erhitzt das erhaltene Öl während 15 Minuten auf 170°. Das Rohprodukt wird durch Chromatographieren an Kieselgel gereinigt, wobei man als Elutionsmittel Methylenchlorid und Gemische aus Methylenchlorid und Essigester (5%, 10%, 15%) verwendet. Nach Umkristallisieren des Rohproduktes aus Essigester/Diäthyläther erhält man reines (S)-1,2,3,11a-Tetrahydro-6-(trifluormethyl)-5H-pyrrolo-[2,1-c][1,4]benzodiazepin-5,11(10H)-dion vom Schmelzpunkt 176—178°.

b) Man versetzt eine Lösung von 9,15 g (32,2 mMol) (S)-1,2,3,11a-Tetrahydro-6-(trifluormethyl)-5H-pyrrolo[2,1-c][1,4]benzodiazepin-5,11(10H)-dion in 30 ml trockenem Dimethylformamid bei −20 bis −10° unter Rühren mit 1,54 g (35,4 mMol) Natriumhydrid (55-proz. Öldispersion), rührt noch während 1 Stunde in obigem Temperaturbereich und tropft dann bei −40° 5,3 ml (35,4 mMol) Diäthylchlorphosphat dazu.

Inzwischen löst man 3,97 g (35,4 mMol) Kalium-t-butylat in 9 ml trockenem Dimethylformamid, kühlt in einem Aceton/Trockeneisbad ab und versetzt mit 4,99 g (35,4 mMol) Isocyanessigsäure-t-butylester. Die erhaltene Lösung tropft man bei −20° zum obigen Reaktionsgemisch. Man läßt auf 10° erwärmen, neutralisiert mit 2,0 ml Eisessig, gießt auf 150 ml Wasser und extrahiert dreimal mit Methylenchlorid. Man wäscht die Methylenchloridlösung einmal mit Wasser, trocknet über Magnesiumsulfat, dampft ein und chromatographiert das erhaltene Rohprodukt unter Eluieren mit Essigester an Kieselgel. Die anschließende Kristallisation aus Diäthyläther liefert t-Butyl-(S)-11,12,13,13a-tetrahydro-9-oxo-8-(trifluormethyl)-9H-imidazo[1,5-a]pyrrolo[2,1-c][1,4]benzodiazepin-1-carboxylat vom Schmelzpunkt 201—203°.

## Beispiel 14

a) Ausgehend von 3-Äthyl-anilin erhält man nach der Sandmeyer'schen Isatinsynthese [T. Sandmeyer, Helv.2, 234 (1919)] 4-Äthyl-isatin. Die Auftrennung der Isomeren nach P. W. Sadler, J. Org. Chemistry 21, 169 (1956), liefert nach Umkristallisieren aus Essigester/Diäthyläther reines 4-Äthylisatin vom Schmelzpunkt 138—140°.

b) Man suspendiert 20 g (0,114 Mol) 4-Äthyl-isatin in 75 ml 100-proz. Essigsäure, versetzt portionenweise mit 26 g (0,137 Mol) m-Chlorperbenzoesäure, wobei die Reaktionstemperatur 50° nicht überschreitet, und rührt dann noch während 15 Minuten bei dieser Temperatur. Das Gemisch wird auf Eiswasser gegossen und filtriert. Das erhaltene Rohprodukt nimmt man in Essigester auf und extrahiert vorsichtig mit einem Gemisch aus 2N-Natronlauge und Eis. Nach Trocknen über Magnesiumsulfat und Einengen der Essigesterphase erhält man rohes 6-Äthyl-isatosäureanhydrid, das nach Umkristallisieren aus Essigester bei 202—204° schmilzt.

c) Man suspendiert 7,65 g (0,04 Mol) 6-Äthyl-isatosäureanhydrid und 4,6 g (0,04 Mol) L-Prolin in 40 ml Dimethylsulfoxid, erhitzt während 2,5 Stunden auf 70°, entfernt das Lösungsmittel im Hochvakuum und erhitzt das erhaltene Öl während 15 Minuten auf 170°. Das Rohprodukt wird durch Chromatographieren an Kieselgel unter Eluieren mit Chloroform gereinigt. Man erhält amorphes (S)-6-Äthyl-1,2,3,11a-tetrahydro-5H-pyrrolo[2,1-c][1,4]benzodiazepin-5,11(10H)-dion.

d) Man versetzt eine Lösung von 7,45 g (30,5 mMol) (S)-6-Äthyl-1,2,3,11a-tetrahydro-5H-pyrrolo[2,1-c][1,4]benzodiazepin-5,11(10H)-dion in 25 ml trockenem Dimethylformamid bei — 20° unter Rühren mit 1,46 g (33,55 mMol) Natriumhydrid (55-proz. Öldispersion), rührt während 1 Stunde bei obiger Temperatur und tropft anschließend bei −45° 5,0 ml (33,55 mMol) Diäthylchlorphosphat dazu.

Inzwischen löst man 3,76 g (33,55 mMol) Kalium-t-butylat in 9 ml trockenem Dimethylformamid, kühlt in einem Aceton/Trockeneisbad ab, versetzt mit 4,7 g (33,55 mMol) Isocyanessigsäure-t-butylester und gibt die erhaltene Lösung bei −25° tropfenweise zum obigen Reaktionsgemisch. Man rührt während 15 Minuten ohne Kühlung, neutralisiert mit 1,9 ml Eisessig, gießt auf 100 ml Wasser und extrahiert dreimal mit Methylenchlorid. Die organischen Auszüge werden zweimal mit Wasser und einmal mit gesättigter Natriumchloridlösung gewaschen, über Magnesiumsulfat getrocknet und eingedampft. Das erhaltene Rohprodukt chromatographiert man unter Eluieren mit Essigester, das 50% n-Hexan enthält, an Kieselgel. Durch Umkristallisieren aus Essigester/n-Hexan erhält man t-Butyl-(S)-8-äthyl-11,12,13,13a-tetrahydro-9-oxo-9H-imidazo[1,5-a]pyrrolo[2,1-c][1,4]benzodiazepin-1-carboxylat vom Schmelzpunkt 155—156°.

## Beispiel 15

Man löst 3,37 g (30 mMol) Kalium-t-butylat in 50 ml Dimethylformamid, kühlt in einem Aceton/Trockeneisbad ab und leitet 2,7 g (56 mMol) Methylmercaptan ein. Zu dieser Lösung gibt man 10 g (26,75 mMol) t-Butyl-(S)-8-chlor-11,12,13,13a-tetrahydro-9-oxo-9H-imidazo[1,5-a]pyrrolo[2,1-c][1,4]-benzodiazepin-1-carboxylat und erwärmt dann während 1 Stunde auf 80°. Anschließend wird die Lösung auf 250 ml Wasser gegossen. Das ausgefallene Produkt wird abgenutscht, mit Wasser gewaschen und im Vakuum getrocknet. Man erhält dabei t-Butyl-(S)-11,12,13,13a-tetrahydro-8-(methylthio)-9-oxo-9H-imidazo[1,5-a]pyrrolo[2,1-c][1,4]benzodiazepin-1-carboxylat vom Schmelzpunkt 236°.

## Beispiel 16

Man löst 4,0 g (10,38 mMol) t-Butyl-(S)-11,12,13,13a-tetrahydro-8-(methylthio)-9-oxo-9H-imidazo[1,5-a]pyrrolo[2,1-c][1,4]benzodiazepin-1-carboxylat in 25 ml Methylenchlorid und versetzt bei Raumtemperatur portionsweise mit 2,0 g (ca. 10,4 mMol) ca. 90-proz. m-Chlorperbenzoesäure und läßt über Nacht bei Raumtemperatur stehen. Dann gießt man die Lösung auf 2N-Natronlauge, trennt die Methylenchloridphase ab, trocknet diese mit Magnesiumsulfat und dampft ein. Nach Umkristallisieren

aus Essigester/Hexan erhält man t-Butyl-(S)-11,12,13,13a-tetrahydro-8-(methylsulfinyl)-9-oxo-9H-imidazo[1,5-a]pyrrolo[2,1-c][1,4]benzodiazepin-1-carboxylat vom Schmelzpunkt 224—225° als Diastereoisomerengemisch.

## Beispiel 17

Man löst 4 g (10,38 mMol) t-Butyl-(S)-11,12,13,13a-tetrahydro-8-(methylthio)-9-oxo-9H-imidazo[1,5-a]pyrrolo[2,1-c][1,4]benzodiazepin-1-carboxylat in 50 ml Methylenchlorid und versetzt bei Raumtemperatur portionenweise mit 4,0 g (ca. 20,8 mMol) ca. 90-proz. m-Chlorperbenzoesäure. Anschließend erwärmt man während 2,5 Stunden unter Rückfluß zum Sieden, gießt dann auf 2N-Natronlauge und trennt die Methylenchloridlösung ab. Man trocknet die organische Phase über Magnesiumsulfat und dampft ein. Nach Umkristallisieren aus Chloroform/Hexan erhält man t-Butyl-(S)-11,12,13,13a-tetrahydro-8-(methylsulfonyl)-9-oxo-9H-imidazo[1,5-a]pyrrolo[2,1-c][1,4]benzodiazepin-1-carboxylat vom Schmelzpunkt 244°.

## Beispiel 18

a) Man erhitzt 4,8 g (0,025 Mol) 6-Methoxy-isatosäureanhydrid und 3,0 g (0,026 Mol) L-Prolin in 40 ml Dimethylsulfoxid während 2 Stunden auf 70°, entfernt das Lösungsmittel im Hochvakuum und erhitzt das erhaltene Öl während 15 Minuten auf 170°. Das Rohprodukt wird nach Behandeln mit Aktivkohle aus Methanol umkristallisiert, wobei man (S)-1,2,3,11a-Tetrahydro-6-methoxy-5H-pyrrolo[2,1-c][1,4]benzodiazepin-5,11(10H)-dion vom Schmelzpunkt 247—251° erhält.
b) Man versetzt eine Suspension von 0,96 g (22 mMol) Natriumhydrid (55-proz. Öldispersion) in 30 ml trockenem Dimethylformamid bei —10° unter Rühren mit 4,7 g (19,1 mMol) (S)-1,2,3,11a-Tetrahydro-6-methoxy-5H-pyrrolo[2,1-c][1,4]benzodiazepin-5,11(10H)-dion, rührt noch während 50 Minuten bei obiger Temperatur und tropft anschließend bei —35° 3,7 ml (22 mMol) Diäthylchlorphosphat dazu.

Inzwischen löst man 2,4 g (22 mMol) Kalium-t-butylat in 6,0 ml trockenem Dimethylformamid, kühlt in einem Aceton/Trockeneisbad ab, versetzt mit 3,1 g (22 mMol) Isocyanessigsäure-t-butylester und tropft die erhaltene Lösung bei —15° zum obigen Reaktionsgemisch. Man rührt noch während 20 Minuten ohne Kühlung, neutralisiert dann mit 1,3 ml Eisessig, gießt auf 80 ml Wasser und extrahiert viermal mit Methylenchlorid. Die organischen Auszüge werden einmal mit Wasser gewaschen, über Magnesiumsulfat getrocknet und eingedampft. Man chromatographiert den Rückstand unter Eluieren mit Essigester, das 5% Methanol enthält, an Kieselgel. Durch Umkristallisieren aus Essigester-/n-Hexan erhält man t-Butyl-(S)-11,12,13,13a-tetrahydro-8-methoxy-9-oxo-9H-imidazo[1,5-a]pyrrolo[2,1-c][1,4]benzodiazepin-1-carboxylat vom Schmelzpunkt 200—201°.

## Beispiel 19

Man versetzt eine Lösung von 7,03 g (30 mMol) (S)-7-Fluor-1,2,3,11a-tetrahydro-5H-pyrrolo[2,1-c][1,4]benzodiazepin-5,11(10H)-dion in 50 ml trockenem Dimethylformamid bei —25° unter Rühren mit 1,51 g (34,5 mMol) Natriumhydrid (55-proz. Öldispersion), rührt während 1 Stunde bei dieser Temperatur und tropft anschließend bei —40° 5,1 ml (34,5 mMol) Diäthylchlorphosphat dazu.

Inzwischen löst man 3,86 g (34,5 mMol) Kalium-t-butylat in 10 ml trockenem Dimethylformamid, kühlt in einem Aceton/Trockeneisbad ab, versetzt mit 4,86 g (34,5 mMol) Isocyanessigsäure-t-butylester und gibt die erhaltene Lösung bei —15° tropfenweise zum obigen Reaktionsgemisch. Man läßt auf 5° erwärmen, neutralisiert mit 3,9 ml Eisessig, gießt auf 250 ml Wasser und extrahiert dreimal mit Methylenchlorid. Die organischen Auszüge werden zweimal mit Wasser gewaschen, über Magnesiumsulfat getrocknet und eingedampft. Das erhaltene Rohprodukt chromatographiert man unter Eluieren mit Essigester an Kieselgel. Nach Umkristallisieren aus Äthanol erhält man t-Butyl-(S)-7-fluor-11,12,13,13a-tetrahydro-9-oxo-9H-imidazo[1,5-a]pyrrolo[2,1-c][1,4]benzodiazepin-1-carboxylat vom Schmelzpunkt 154—155°.

## Beispiel 20

a) Ausgehend von 3-Chlor-4-fluor-anilin erhält man nach der Sandmeyer'schen Isatinsynthese [T. Sandmeyer, Helv. 2, 234 (1919)] 4-Chlor-5-fluor-isatin. Die Auftrennung der Isomeren nach P. W. Sadler, J. Org. Chemistry 21, 169 (1956) liefert nach Umkristallisieren reines 4-Chlor-5-fluor-isatin vom Schmelzpunkt 249—251°.
b) Man suspendiert 7,8 g (0,039 Mol) 4-Chlor-5-fluor-isatin in 50 ml 100-proz. Essigsäure, versetzt mit

0,25 ml konzentrierter Schwefelsäure und tropft dann bei 30° 4,4 ml (0,043 Mol) 30-proz. Wasserstoffperoxid dazu. Das Reaktionsgemisch wird anschließend während 2,5 Stunden auf 70° erhitzt, dann auf 10° abgekühlt und filtriert. Das Rohprodukt wird aus Aceton/Hexan umkristallisiert, wobei man 6-Chlor-5-fluor-isatosäureanhydrid vom Schmelzpunkt 275—278° (Zersetzung) erhält.

c) Man erhitzt 3,3 g (0,015 Mol) 6-Chlor-5-fluor-isatosäureanhydrid und 2 g (0,017 Mol) L-Prolin in 7,5 ml Dimethylformamid während 2 Stunden auf 120°, versetzt nach dem Abkühlen mit 12 ml dest. Wasser und filtriert die ausgefallenen braunen Kristalle ab. Durch Umkristallisieren des Rohproduktes aus Aceton/Hexan erhält man (S)-6-Chlor-7-fluor-1,2,3,11a-tetrahydro-5H-pyrrolo[2,1-c][1,4]benzodiazepin-5,11(10H)-dion vom Schmelzpunkt 217—219°.

d) Man versetzt eine Lösung von 7,16 g (26,6 mMol) (S)-6-Chlor-7-fluor-1,2,3,11a-tetrahydro-5H-pyrrolo[2,1-c][1,4]benzodiazepin-5,11(10H)-dion in 25 ml trockenem Dimethylformamid bei —20 bis —10° unter Rühren mit 1,27 g (29,26 mMol) Natriumhydrid (55-proz. Öldispersion), rührt noch während 1,25 Stunden in obigem Temperaturbereich und tropft dann bei —40° 4,4 ml (29,26 mMol) Diäthylchlorphosphat dazu.

Inzwischen löst man 3,28 g (29,3 mMol) Kalium-t-butylat in 8ml trockenem Dimethylformamid, kühlt in einem Aceton/Trockeneisbad ab, versetzt mit 4,13 g (29,3 mMol) Isocyanessigsäure-t-butylester und tropft die erhaltene Lösung bei —20 bis —10° zum obigen Reaktionsgemisch. Man läßt auf 10° erwärmen, neutralisiert mit 1,7 ml Eisessig, gießt auf 150 ml Wasser und extrahiert dreimal mit Methylenchlorid. Man wäscht die Methylenchloridlösung einmal mit Wasser, trocknet über Magnesiumsulfat, dampft ein und chromatographiert das erhaltene Rohprodukt unter Eluieren mit Essigester an Kieselgel. Durch Kristallisieren aus Diäthyläther erhält man t-Butyl-(S)-8-Chlor-7-fluor-11,12,13,13a-tetrahydro-9-oxo-9H-imidazo[1,5-a]pyrrolo[2,1-c][1,4]benzodiazepin-1-carboxylat vom Schmelzpunkt 211—212°.

Beispiel 21

a) Man erhitzt 37,0 g (187,3 mMol) 6-Chlorisatosäureanhydrid und 24,6 g (187,3 mMol) L-4-Hydroxyprolin in 180 ml Dimethylsulfoxid während 2 Stunden auf 100°, dampft im Hochvakuum zur Trockne ein und erhitzt den erhaltenen Rückstand während 2 Stunden auf 130°. Durch Umkristallisieren aus Äthanol erhält man (2R,11aS)-6-Chlor-1,2,3,11a-tetrahydro-2-hydroxy-5H-pyrrolo[2,1-c][1,4]benzodiazepin-5,11(10H)-dion vom Schmelzpunkt 284—287°.

b) Man versetzt eine Suspension von 4,0 g (15 mMol) (2R,11aS)-6-Chlor-1,2,3,11a-tetrahydro-2-hydroxy-5H-pyrrolo[2,1-c][1,4]benzodiazepin-5,11(10H)-dion in 20 ml Pyridin unter Rühren mit 1,4 ml (18 mMol) Methansulfochlorid, rührt noch während 1,25 Stunden bei Raumtemperatur, dampft die Lösung ein und verteilt den Rückstand zwischen 100 ml Chloroform und 70 ml Wasser. Man wäscht die Chloroformlösung zweimal mit Wasser, trocknet über Magnesiumsulfat und dampft ein. Durch Kristallisieren des erhaltenen Rückstandes aus Äthanol erhält man (2R,11aS)-6-Chlor-2,3,5,10,11,11a-hexahydro-5,11-dioxo-1H-pyrrolo[2,1-c][1,4]benzodiazepin-2-yl-methansulfonat vom Schmelzpunkt 213—215°.

c) Man versetzt eine Lösung von 17,0 g (49,3 mMol) (2R,11aS)-6-Chlor-2,3,5,10,11,11a-hexahydro-5,11-dioxo-1H-pyrrolo[2,1-c][1,4]benzodiazepin-2-yl-methansulfonat in 80 ml trockenem Dimethylformamid mit 4,3 g (98,6 mMol) Natriumhydrid (55-proz. Öldispersion), rührt noch während 2 Stunden bei 45°, gießt dann auf 300 ml Eiswasser, neutralisiert mit 5,6 ml Eisessig und extrahiert viermal mit Methylenchlorid. Die organischen Auszüge werden einmal mit gesättigter Natriumchloridlösung gewaschen, über Magnesiumsulfat getrocknet und eingedampft. Durch Kristallisieren aus Äthanol erhält man (S)-6-Chlor-1,11a-dihydro-5H-pyrrolo[2,1-c][1,4]benzodiazepin-5,11(10H)-dion vom Schmelzpunkt 249—251°.

d) Man versetzt eine Lösung von 8,59 g (34,5 mMol) (S)-6-Chlor-1,11a-dihydro-5H-pyrrolo[2,1-c][1,4]benzodiazepin-5,11(10H)-dion in 35 ml trockenem Dimethylformamid bei —20 bis —10° unter Rühren mit 1,66 g (38 mMol) Natriumhydrid (55-proz. Öldispersion), rührt noch während 1 Stunde in obigem Temperaturbereich und tropft anschließend bei —40° 5,5 ml (38 mMol) Diäthylchlorphosphat dazu.

Inzwischen löst man 4,3 g (38 mMol) Kalium-t-butylat in 7 ml trockenem Dimethylformamid, kühlt in einem Aceton/Trockeneisbad ab, versetzt mit 6,0 g (38 mMol) ca. 90-proz. Isocyanessigsäure-t-butylester und gibt die erhaltene Lösung bei —20° tropfenweise zum obigen Reaktionsgemisch. Man läßt auf 10° erwärmen, neutralisiert mit 2,2 ml Eisessig, gießt auf 200 ml Wasser und extrahiert viermal mit Methylenchlorid. Die Methylenchloridlösung wird einmal mit Wasser gewaschen, über Magnesiumsulfat getrocknet und eingedampft. Man löst den Rückstand in heißem Essigester, läßt über Nacht im Eisschrank stehen und nutscht das ausgefallene Material unter Nachwaschen mit kaltem Essigester ab. Umkristallisieren des Rohproduktes aus Äthanol liefert t-Butyl-(S)-8-chlor-13,13a-dihydro-9-oxo-9H-imidazo[1,5-a]pyrrolo[2,1-c][1,4]benzodiazepin-1-carboxylat vom Schmelzpunkt 235—237°.

Beispiel 22

a) Man erhitzt 13,0 g (128,4 mMol) L-Azetidin-2-carbonsäure und 22,7 g (128,4 mMol) 6-Methyl-isatosäureanhydrid in 150 ml Dimethylsulfoxid während 3 Stunden auf 95°, dampft im Hochvakuum zur Trockne ein und erhitzt den erhaltenen Rückstand während 2,25 Stunden auf 140°. Durch Kristallisieren aus Essigester erhält man (S)-1,10a-Dihydro-5-methyl-2H-azeto[2,1-c][1,4]benzodiazepin-4,10-(9H)-dion vom Schmelzpunkt 159—160°.

b) Man versetzt eine Lösung von 13,8 g (63,8 mMol) (S)-1,10a-Dihydro-5-methyl-2H-azeto[2,1-c][1,4]benzodiazepin-4,10(9H)-dion in 55 ml trockenem Dimethylformamid bei —20 bis —10° unter Rühren mit 3,06 g (70,2 mMol) Natriumhydrid (55-proz. Öldispersion), rührt noch während 40 Minuten bei obiger Temperatur und tropft anschließend bei —35° 10,5 ml (70,2 mMol) Diäthylchlorphosphat dazu.

Inzwischen löst man 7,88 g (70,2 mMol) Kalium-t-butylat in 12 ml trockenem Dimethylformamid, kühlt in einem Aceton/Trockeneisbad ab, versetzt mit 11 g (70,2 mMol) ca. 90-proz. Isocyanessigsäure-t-butylester und tropft die erhaltene Lösung bei —20 bis —15° zum obigen Reaktionsgemisch. Man läßt auf 0° erwärmen, neutralisiert mit 4 ml Eisessig, gießt auf 300 ml Wasser und extrahiert dreimal mit Methylenchlorid. Die Methylenchloridlösung wird zweimal mit Wasser gewaschen, über Magnesiumsulfat getrocknet und eingedampft. Das erhaltene Rohprodukt chromatographiert man unter Eluieren mit Essigester, das 50% n-Hexan enthält, an Kieselgel. Nach Umkristallisieren aus Essigester/n-Hexan erhält man t-Butyl-(S)-12,12a-dihydro-8-methyl-9-oxo-9H,11H-azeto[2,1-c]imidazo[1,5-a][1,4]benzodiazepin-1-carboxylat vom Schmelzpunkt 191—192°.

Beispiel 23

11,0 g (47,5 mMol) 6-(Trifluormethyl)isatosäureanhydrid und 4,8 g (47,5 mMol) L-Azetidin-2-carbonsäure werden in 50 ml Dimethylsulfoxid während 45 Minuten auf 105° erhitzt. Anschließend dampft man im Hochvakuum zur Trockne ein und erhitzt den erhaltenen Rückstand während 2 Stunden auf 150°. Das Rohprodukt reinigt man durch Chromatographieren an Kieselgel, wobei man als Elutionsmittel Methylenchlorid, das 7% Essigester enthält, verwendet.

7,3 g des erhaltenen Öles nimmt man in 30 ml trockenem Dimethylformamid auf, versetzt die Lösung bei —20° mit 1,26 g (29 mMol) Natriumhydrid (55-proz. Öldispersion), rührt noch während 50 Minuten bei obiger Temperatur und tropft dann bei —25° eine Lösung von 7,38 g (29 mMol) Dimorpholinophosphonsäurechlorid in 10 ml trockenem Dimethylformamid dazu.

Inzwischen löst man 3,25 g (29 mMol) Kalium-t-butylat in 8 ml trockenem Dimethylformamid, kühlt in einem Aceton/Trockeneisbad ab, versetzt mit 4,09 g (29 mMol) Isocyanessigsäure-t-butylester und gibt die erhaltene Lösung bei —20° tropfenweise zum obigen Reaktionsgemisch. Man rührt noch während 20 Stunden ohne Kühlung, neutralisiert mit 1,6 ml Eisessig, gießt auf 100 ml Wasser und extrahiert viermal mit Methylenchlorid. Man wäscht die Methylenchloridlösung einmal mit Wasser und einmal mit gesättigter Natriumchloridlösung, trocknet über Magnesiumsulfat und dampft ein. Das erhaltene Rohprodukt chromatographiert man unter Eluieren mit Essigester/n-Hexan (3 : 2) und Essigester an Kieselgel. Durch Umkristallisieren aus Essigester erhält man t-Butyl-(S)-12,12a-dihydro-9-oxo-8-(trifluormethyl)-9H,11H-azeto[2,1-c]imidazo[1,5-a][1,4]benzodiazepin-1-carboxylat mit einem Zersetzungspunkt von 217—219°.

Beispiel 24

a) Man rührt 36 g (0,26 Mol) 3-Aminopyridin-2-carbonsäure, 46,6 g (0,29 Mol) N,N'-Carbonyldiimidazol und 200 ml Dimethylformamid bei Raumtemperatur bis zur Beendigung der Kohlendioxidentwicklung, versetzt dann mit 26,3 g (0,26 Mol) Triäthylamin und 43,1 g (0,26 Mol) L-Prolinmethylester-hydrochlorid und rührt noch während 1,5 Stunden bei Raumtemperatur. Anschließend gießt man die Reaktionslösung auf Wasser, extrahiert viermal mit Chloroform, trocknet die vereinigten Chloroformauszüge über Magnesiumsulfat und dampft diese im Vakuum ein. Den öligen Rückstand versetzt man mit 500 ml Eisessig, erhitzt während 1 Stunde unter Rückfluß zum Sieden und dampft dann zur Trockne ein. Durch Umkristallisieren aus Äthanol erhält man 6a,7,8,9-Tetrahydro-6H-pyrido[3,2-e]pyrrolo[1,2-a][1,4]diazepin-6,11(5H)-dion vom Schmelzpunkt 249—250°.

b) Man versetzt eine Lösung von 6,33 g (29,1 mMol) 6a,7,8,9-Tetrahydro-6H-pyrido[3,2-e]pyrrolo[1,2-a][1,4]diazepin-6,11(5H)-dion in 20 ml trockenem Dimethylformamid bei 0° unter Rühren mit 1,4 g (29,1 mMol) Natriumhydrid (50-proz. Öldispersion), rührt noch während 1 Stunde bei obiger Temperatur und tropft dann bei —30° 4,2 ml (29,1 mMol) Diäthylchlorphosphat dazu.

Inzwischen löst man 3,3 g (29,1 mMol) Kalium-t-butylat in 5 ml trockenem Dimethylformamid, kühlt in einem Aceton/Trockeneisbad ab, versetzt mit 4,1 g (29,1 mMol) Isocyanessigsäure-t-butylester und

tropft die erhaltene Lösung bei −20 bis −10° zum obigen Reaktionsgemisch. Man rührt noch während 1 Stunde ohne Kühlung, neutralisiert dann mit 1,7 ml Eisessig, gießt auf 200 ml Wasser und extrahiert dreimal mit Chloroform. Man wäscht die Chloroformlösung dreimal mit Wasser, trocknet über Magnesiumsulfat und dampft ein. Den erhaltenen Rückstand chromatographiert man unter Eluieren mit Chloroform/Methanol (9 : 1) an Kieselgel und kristallisiert das erhaltene Material anschließend aus Essigester um. Man erhält t-Butyl-11,12,13,13a-tetrahydro-9-oxo-9H-imidazo[5,1-c]pyrido[3,2-e]pyrrolo[1,2-a][1,4]diazepin-1-carboxylat vom Schmelzpunkt 230—231°.

Beispiel 25

a) Man erhitzt 13,8 g (0,1 Mol) 4-Aminopyridin-3-carbonsäure, 24,3 g (0,15 Mol) N,N'-Carbonyldiimidazol und 130 ml Dimethylformamid während 4 Stunden auf 55°, versetzt die erhaltene Lösung mit 10,2 g Triäthylamin und 16,5 g (0,1 Mol) L-Prolinmethylester-hydrochlorid, erhitzt noch während 3 Stunden auf 85° und dampft dann im Hochvakuum ein. Zum Rückstand gibt man 125 ml Eisessig, erhitzt während 0,5 Stunden unter Rückfluß zum Sieden und dampft ein. Den Rückstand nimmt man in Chloroform auf, wäscht die erhaltene Lösung mit Wasser, trocknet sie über Magnesiumsulfat und dampft ein. Durch Chromatographieren an Kieselgel mit Essigester/Methanol als Eluationsmittel erhält man (S)-6a,7,8,9-Tetrahydro-6H-pyrido[4,3-a]pyrrolo[1,2-a][1,4]diazepin-6,11(5H)-dion vom Schmelzpunkt 266—268°.

b) Man versetzt eine Lösung von 7,15 g (33 mMol) (S)-6a,7,8,9-Tetrahydro-6H-pyrido[4,3-e]pyrrolo[1,2-a][1,4]diazepin-6,11(5H)-dion in 30 ml trockenem Dimethylformamid bei −10° unter Rühren mit 1,53 g (35 mMol) Natriumhydrid (55-proz. Öldispersion), rührt noch während 30 Minuten bei obiger Temperatur und tropft dann bei −30° 5 ml (35 mMol) Diäthylchlorphosphat dazu.

Inzwischen löst man 3,92 g (35 mMol) Kalium-t-butylat in 6 ml trockenem Dimethylformamid, kühlt in einem Aceton/Trockeneisbad ab, versetzt mit 4,8 g (34 mMol) Isocyanessigsäure-t-butylester und gibt die erhaltene Lösung bei −20 bis −10° tropfenweise zum obigen Reaktionsgemisch. Man läßt auf Raumtemperatur erwärmen, neutralisiert mit 3,5 ml Eisessig, gießt auf 300 ml Wasser und extrahiert dreimal mit Chloroform. Die organischen Auszüge werden dreimal mit Wasser gewaschen, über Magnesiumsulfat getrocknet und eingedampft. Durch Kristallisieren aus Essigester erhält man t-Butyl-11,12,13,13a-tetrahydro-9-oxo-9H-imidazo[5,1-c]pyrido[4,3-e]pyrrolo[1,2-a][1,4]diazepin-1-carboxylat vom Schmelzpunkt 217—218°.

Beispiel 26

a) Man versetzt 15 g (43,4 mMol) Äthyl-(S)-8-chlor-11,12,13,13a-tetrahydro-9-oxo-9H-imidazo[1,5-a]pyrrolo[2,1-c][1,4]benzodiazepin-1-carboxylat und 1,85 g (46,3 mMol) Natriumhydroxid mit 60 ml Äthanol und 10 ml Wasser erhitzt dann während 45 Minuten unter Rückfluß zum Sieden, destilliert anschließend das Äthanol im Vakuum ab, versetzt schließlich mit 46,5 ml 1N-Salzsäure und läßt während 2 Stunden im Eisbad stehen. Das ausgefallene Material wird abgenutscht, mit Wasser gewaschen und bis zur Gewichtskonstanz getrocknet. Man erhält (S)-8-Chlor-11,12,13,13a-tetrahydro-9-oxo-9H-imidazo[1,5-a]pyrrolo[2,1-c][1,4]benzodiazepin-1-carbonsäure mit einem Zersetzungspunkt von 265°.

b) Man rührt 9,54 g (30 mMol) (S)-8-Chlor-11,12,13,13a-tetrahydro-9-oxo-9H-imidazo[1,5-a]pyrrolo[2,1-c][1,4]benzodiazepin-1-carbonsäure und 6,32 g (39 mMol) N,N'-Carbonyldiimidazol in 50 ml trockenem Dimethylformamid während 1 Stunde bei Raumtemperatur und während 1 Stunde bei 50°. Anschließend gießt man auf ca. 300 ml Wasser, nutscht das ausgefallene Material ab, wäscht es mit Wasser und trocknet es bis zur Gewichtskonstanz. Man erhält (S)-1-[(8-Chlor-11,12,13,13a-tetrahydro-9-oxo-9H-imidazo[1,5-a]pyrrolo[2,1-c][1,4]benzodiazepin-1-yl)carbonyl]-imidazol vom Schmelzpunkt 240—241,5°.

c) Man versetzt eine Suspension von 1,1 g (3 mMol) (S)-1-[(8-chlor-11,12,13,13a-tetrahydro-9-oxo-9H-imidazo[1,5-a]pyrrolo[2,1-c][1,4]benzodiazepin-1-yl)carbonyl]imidazol in 10 ml trockenem Dimethylformamid unter Rühren mit 0,34 g (3 mMol) Kalium-t-butylat, rührt noch während 30 Minuten bei Raumtemperatur, gießt dann auf 200 ml Wasser und extrahiert dreimal mit Chloroform. Man wäscht die Chloroformlösung dreimal mit Wasser, trocknet über Magnesiumsulfat und dampft ein. Nach Kristallisieren aus Essigester erhält man t-Butyl-8-chlor-11,12,13,13a-tetrahydro-9-oxo-9H-imidazo[1,5-a]pyrrolo[2,1-c][1,4]benzodiazepin-1-carboxylat vom Schmelzpunkt 223—225°.

Beispiel 27

a) 57,3 g (219 mMol) (S)-1,2,3,11a-Tetrahydro-7-nitro-5H-pyrrolo[2,1-c][1,4]benzodiazepin-5,11(10H)-dion werden in 1,3 l Methanol über 3 g 10-proz. Palladiumkohle bei Raumtemperatur und

Normaldruck hydriert. Nach beendeter Wasserstoffaufnahme wird vom Katalysator abgenutscht; man dampft das Filtrat im Vakuum ein und kristallisiert den erhaltenen Rückstand aus Isopropanol um. Man erhält dabei (S)-7-Amino-1,2,3,11a-tetrahydro-5H-pyrrolo[2,1-c][1,4]benzodiazepin-5,11(10H)-dion vom Schmelzpunkt 237,5—238,5°.

b) Man versetzt unter Rühren eine Lösung von 4,61 g (20 mMol) (S)-7-Amino-1,2,3,11a-tetrahydro-5H-pyrrolo[2,1-c][1,4]benzodiazepin-5,11(10H)-dion in 25 ml trockenem Dimethylformamid unter Eis/Methanol-Kühlung mit 0,96 g (20 mMol) Natriumhydrid (55-proz. Öldispersion), rührt dann noch während 20 Minuten bei Raumtemperatur und tropft anschließend bei —30° eine Lösung von 5,1 g (20 mMol) Dimorpholinophosphonsäurechlorid in 15 ml trockenem Dimethylformamid dazu.

Inzwischen löst man 2,47 g (20 mMol) Kalium-t-butylat in 5 ml trockenem Dimethylformamid, kühlt in einem Aceton/Trockeneisbad ab, versetzt mit 2,82 g (20 mMol) Isocyanessigsäure-t-butylester und gibt die erhaltene Lösung bei −20° tropfenweise zum obigen Reaktionsgemisch. Man rührt noch während 30 Minuten ohne Kühlung, neutralisiert mit 1,1 ml Eisessig, gießt auf 300 ml Wasser und extrahiert dreimal mit Chloroform. Man wäscht die Chloroformlösung dreimal mit Wasser, trocknet sie über Magnesiumsulfat und dampft ein. Man chromatographiert den erhaltenen Rückstand unter Eluieren mit Chloroform, das 3,6% Methanol enthält, an Kieselgel. Durch Kristallisieren des erhaltenen Materials aus Essigester erhält man t-Butyl-(S)-7-amino-11,12,13,13a-tetrahydro-9-oxo-9H-imidazo[1,5-a]pyrrolo[2,1-c][1,4]benzodiazepin-1-carboxylat vom Schmelzpunkt 223—224°.

## Beispiel 28

Man rührt 1,5 g (4,2 mMol) t-Butyl-(S)-7-amino-11,12,13,13a-tetrahydro-9-oxo-9H-imidazo[1,5-a]pyrrolo[2,1-c][1,4]benzodiazepin-1-carboxylat und 0,783 g (4,4 mMol) N-Bromsuccinimid in 15 ml Dimethylformamid während 45 Minuten bei Raumtemperatur, gießt anschließend auf 250 ml Wasser und extrahiert dreimal mit Chloroform. Man wäscht die Chloroformlösung dreimal mit Wasser, trocknet sie über Magnesiumsulfat und dampft ein. Den erhaltenen Rückstand chromatographiert man unter Eluieren mit Chloroform, das 4% Methanol enthält, an Kieselgel. Umkristallisieren des erhaltenen Materials aus Essigester liefert t-Butyl-(S)-7-amino-8-brom-11,12,13,13a-tetrahydro-9-oxo-9H-imidazo[1,5-a]pyrrolo[2,1-c][1,4]benzodiazepin-1-carboxylat vom Schmelzpunkt 245—246°.

## Beispiel 29

Man erhitzt 380 mg (0,9 mMol) t-Butyl-(S)-7-amino-8-brom-11,12,13,13a-tetrahydro-9-oxo-9H-imidazo[1,5-a]pyrrolo[2,1-c][1,4]benzodiazepin-1-carboxylat und 109 mg (1,05 mMol) t-Butylnitrit in 10 ml Tetrahydrofuran über Nacht unter Rückfluß zum Sieden und dampft anschließend im Vakuum zur Trockne ein. Den erhaltenen Rückstand chromatographiert man unter Eluieren mit Essigester an Kieselgel. Durch Kristallisieren aus Essigester erhält man schließlich t-Butyl-(S)-8-brom-11,12,13,13a-tetrahydro-9-oxo-9H-imidazo[1,5-a]pyrrolo[2,1-c][1,4]benzodiazepin-1-carboxylat vom Schmelzpunkt 204—205°.

t-Butyl-(S)-8-chlor-11,12,13,13a-tetrahydro-9-oxo-9H-imidazo[1,5-a]pyrrolo[2,1-c][1,4]benzodiazepin-1-carboxylat kann wie in den nachfolgenden Beispielen A bis C angegeben als Wirkstoff zur Herstellung von pharmazeutischen Präparaten verwendet werden:

## Beispiel A

Es werden in üblicher Weise Tabletten folgender Zusammensetzung hergestellt:

| | mg/Tablette |
|---|---|
| Wirkstoff | 5 |
| Milchzucker | 45 |
| Maisstärke | 15 |
| Mikrokristalline Cellulose | 34 |
| Magnesiumstearat | 1 |
| Tablettengewicht | 100 |

## Beispiel B

Es werden Kapseln folgender Zusammensetzung hergestellt:

| | | mg/Kapsel |
|---|---|---|
| Wirkstoff | | 10 |
| Milchzucker | | 155 |
| Maisstärke | | 30 |
| Talk | | 5 |
| | Kapselfüllgewicht | 200 |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

## Beispiel C

Es werden Suppositorien folgender Zusammensetzung hergestellt:

| | | mg/Supp. |
|---|---|---|
| Wirkstoff | | 15 |
| Suppositorienmasse | | 1285 |
| | Total | 1300 |

Die Suppositorienmasse wird in einem Glas- oder Stahlgefäß geschmolzen, gründlich vermengt und auf 45° abgekühlt. Hierauf gibt man den fein pulverisierten Wirkstoff zu und rührt, bis er vollständig dispergiert ist. Man gießt die Mischung in Suppositorienformen geeigneter Größe, läßt abkühlen, nimmt dann die Suppositorien aus den Formen und verpackt sie einzeln in Wachspapier oder Metallfolien.

Die nachfolgend aufgeführten Verbindungen der Formel I können anstelle von t-Butyl-(S)-8-chlor-11,12,13,13a-tetrahydro-9-oxo-9H-imidazo[1,5-a]pyrrolo[2,1-c][1,4]benzodiazepin-1-carboxylat wie in den obigen Beispielen A bis C angegeben als Wirkstoffe verwendet werden:

t-Butyl-(S)-8-chlor-12,12a-dihydro-9-oxo-9H,11H-azeto-[2,1-c]imidazo[1,5-a][1,4]-benzodiazepin-1-carboxylat,
t-Butyl-(S)-11,12,13,13a-tetrahydro-8-methyl-9-oxo-9H-imidazo[1,5-a]-pyrrolo[2,1-c][1,4]benzodiazepin-1-carboxylat,
t-Butyl-(S)-8-brom-11,12,13,13a-tetrahydro-9-oxo-9H-imidazo[1,5-a]pyrrolo-[2,1-c][1,4]benzodiazepin-1-carboxylat,
t-Butyl-(S)-11,12,13,13a-tetrahydro-8-jod-9-oxo-9H-imidazo[1,5-c]pyrrolo-[2,1-c][1,4]benzodiazepin-1-carboxylat,
t-Butyl-(S)-11,12,13,13a-tetrahydro-9-oxo-8-(trifluormethyl)-9H-imidazo[1,5-a]-pyrrolo[2,1-c][1,4]benzodiazepin-1-carboxylat,
t-Butyl-(S)-8-äthyl-11,12,13,13a-tetrahydro-9-oxo-9H-imidazo[1,5-a]pyrrolo-[2,1-c][1,4]benzodiazepin-1-carboxylat,
t-Butyl-(S)-8-chlor-7-fluor-11,12,13,13a-tetrahydro-9-oxo-9H-imidazo[1,5-a]-pyrrolo[2,1-c][1,4]benzodiazepin-1-carboxylat,
t-Butyl-(S)-12,12a-dihydro-8-methyl-9-oxo-9H,11H-azeto[2,1-c]imidazo[1,5-a][1,4]-benzodiazepin-1-carboxylat und
t-Butyl-(S)-12,12a-dihydro-9-oxo-8-(trifluormethyl)-9H,11H-azeto[2,1-c]imidazo-[1,5-a][1,4]benzodiazepin-1-carboxylat.

**Patentansprüche für die Vertragsstaaten: BE, CH, DE, FR, GB, IT, LI, LU, NL, SE**

1. Imidazodiazepine der allgemeinen Formel

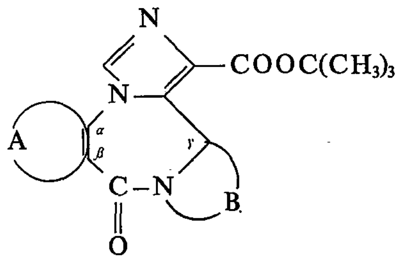

(I)

worin A zusamen mit den beiden mit $\alpha$ und $\beta$ bezeichneten Kohlenstoffatomen die Gruppe

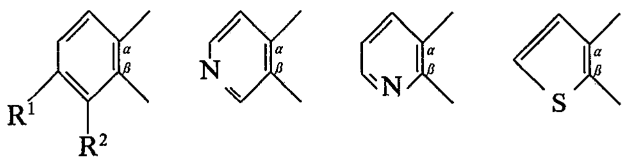

(a) (b) (c) oder (d)

B Dimethylen, Trimethylen oder Propenylen, $R^1$ Wasserstoff, Halogen, Trifluormethyl, Amino, Nitro, Cyano oder niederes Akyl und $R^2$ Wasserstoff, Halogen, Trifluormethyl, Amino, Nitro, Cyano, niederes Alkyl, niederes Alkyloxy, niederes Alkylthio, niederes Alkylsulfinyl oder niederes Alkylsulfonyl bedeuten, das mit $\gamma$ bezeichnete Kohlenstoffatom die (S)- oder (R,S)-Konfiguration aufweist und die mit nieder bezeichneten Reste höchstens 7 Kohlenstoffatome besitzen
und pharmazeutisch annehmbare Säureadditionssalze davon.

2. Verbindungen gemäß Anspruch 1, dadurch gekennzeichnet, daß A die in Anspruch 1 definierte Gruppe (a), einer der Reste $R^1$ und $R^2$ Wasserstoff und der andere Nitro oder Cyano und B Dimethylen, Trimethylen oder Propenylen bedeuten, und das mit $\gamma$ bezeichnete Kohlenstoffatom die (S)- oder (R,S)-Konfiguration aufweist.

3. Verbindungen gemäß Anspruch 1, dadurch gekennzeichnet, daß A die in Anspruch 1 definierte Gruppe (a) oder (d), B Dimethylen, $R^1$ Wasserstoff, Halogen oder Trifluormethyl und $R^2$ Wasserstoff, Halogen, Trifluormethyl oder niederes Alkyl bedeuten, und das mit $\gamma$ bezeichnete Kohlenstoffatom die (S)- oder (R,S)-Konfiguration aufweist.

4. Verbindungen gemäß Anspruch 1, dadurch gekennzeichnet, daß A die in Anspruch 1 definierte Gruppe (a) oder (d), B Trimethylen oder Propenylen, $R^1$ Wasserstoff, Halogen oder Trifluormethyl und $R^2$ Wasserstoff, Halogen, Trifluormethyl oder niederes Alkyl bedeuten, und das mit $\gamma$ bezeichnete Kohlenstoffatom die (S)- oder (R,S)-Konfiguration aufweist.

5. Verbindungen gemäß Anspruch 1, dadurch gekennzeichnet, daß A die in Anspruch 1 definierte Gruppe (b) oder (c) und B Dimethylen, Trimethylen oder Propenylen bedeuten, und das mit $\gamma$ bezeichnete Kohlenstoffatom die (S)- oder (R, S)-Konfiguration aufweist.

6. Verbindungen gemäß Anspruch 1, dadurch gekennzeichnet, daß A die in Anspruch 1 definierte Gruppe (a), B Dimethylen, Trimethylen oder Propenylen, und entweder $R^1$ niederes Alkyl und $R^2$ Wasserstoff, Halogen, Trifluormethyl, Nitro, Cyano oder niederes Alkyl oder einer der Reste $R^1$ und $R^2$ Nitro oder Cyano und der andere Halogen, Trifluormethyl, Nitro, Cyano oder niederes Alkyl bedeuten, und das mit $\gamma$ bezeichnete Kohlenstoffatom die (S)- oder (R,S)-Konfiguration aufweist.

7. Verbindungen gemäß Anspruch 1, dadurch gekennzeichnet, daß A die in Anspruch 1 definierte Gruppe (a), (b), (c) oder (d), $R^1$ Wasserstoff, Amino oder Halogen und $R^2$ Wasserstoff, Halogen, Trifluormethyl, niederes Alkyl, Cyano, Nitro, Amino, niederes Alkyloxy, niederes Alkylthio, niederes Alkylsulfinyl oder niederes Alkylsulfonyl bedeuten.

8. Verbindungen gemäß Anspruch 7, dadurch gekennzeichnet, daß $R^1$ Wasserstoff oder Halogen und $R^2$ Halogen, Trifluormethyl oder niederes Alkyl bedeuten.

9. Verbindungen gemäß einem der Ansprüche 1, 7 und 8, dadurch gekennzeichnet, daß B Dimethylen oder Trimethylen bedeutet.

10. Verbindungen gemäß einem der Ansprüche 1 und 7 bis 9, dadurch gekennzeichnet, daß das mit $\gamma$ bezeichnete Kohlenstoffatom die (S)-Konfiguration aufweist.

11. t-Butyl-(S)-12,12a-dihydro-9-oxo-8-(trifluormethyl)-9H-11H-azeto[2,1-c]imidazo[1,5-a][1,4]benzodiazepin-1-carboxylat.

12. t-Butyl-(S)-12,12a-dihydro-8-methyl-9-oxo-9H,11H-azeto[2,1-c]imidazo[1,5-a][1,4]benzodiazepin-1-carboxylat.

13. t-Butyl-(S)-8-chlor-7-fluor-11,12,13,13a-tetrahydro-9-oxo-9H-imidazo[1,5-a]pyrrolo[2,1-c][1,4]-benzodiazepin-1-carboxylat.

14. t-Butyl-(S)-8-chlor-11,12,13,13a-tetrahydro-9-oxo-9H-imidazo[1,5-a]pyrrolo[2,1-c][1,4]benzodiazepin-1-carboxylat.

15. t-Butyl-(S)-8-äthyl-11,12,13,13a-tetrahydro-9-oxo-9H-imidazo[1,5-a]pyrrolo[2,1-c][1,4]benzodiazepin-1-carboxylat.

16. t-Butyl-(S)-11,12,13,13a-tetrahydro-9-oxo-8-(trifluormethyl)-9H-imidazo[1,5-a]pyrrolo[2,1-c][1,4]-benzodiazepin-1-carboxylat.

17. t-Butyl-(S)-8-brom-11,12,13,13a-tetrahydro-9-oxo-9H-imidazo[1,5-a]pyrrolo[2,1-c][1,4]benzodiazepin-1-carboxylat.

18. t-Butyl-(S)-11,12,13,13a-tetrahydro-8-jod-9-oxo-9H-imidazo[1,5-a]pyrrolo[2,1-c][1,4]benzodiazepin-1-carboxylat.

19. t-Butyl-(S)-8-chlor-12,12a-dihydro-9-oxo-9H,11H-azeto[2,1-c]imidazo[1,5-a][1,4]benzodiazepin-1-carboxylat.

20. t-Butyl-(S)-11,12,13,13a-tetrahydro-8-methyl-9-oxo-9H-imidazo[1,5-a]pyrrolo[2,1-c][1,4]benzodiazepin-1-carboxylat.

21. t-Butyl-8-chlor-11,13a-dihydro-9-oxo-9H-imidazo[1,5-a]pyrrolo[2,1-c][1,4]benzodiazepin-1-carboxylat,

t-Butyl-(S)-10,11,12,12a-tetrahydro-8-oxo-8H-imidazo[5,1-c]pyrrolo[1,2-a]thieno-[3,2-e][1,4]diazepin-1-carboxylat,

t-Butyl-(S)-11,12,13,13a-tetrahydro-9-oxo-9H-imidazo[1,5-a]pyrrolo[2,1-c][1,4]-benzodiazepin-1-carboxylat,

t-Butyl-(S)-7-chlor-11,12,13,13a-tetrahydro-9-oxo-9H-imidazo[1,5-a]pyrrolo-[2,1-c][1,4]benzodiazepin-1-carboxylat,

t-Butyl-(S)-8-amino-11,12,13,13a-tetrahydro-9-oxo-9H-imidazo[1,5-a]pyrrolo-[2,1-c][1,4]benzodiazepin-1-carboxylat,

t-Butyl-(S)-11,12,13,13a-tetrahydro-8-nitro-9-oxo-9H-imidazo[1,5-a]pyrrolo-[2,1-c][1,4]benzodiazepin-1-carboxylat,

t-Butyl-(S)-8-cyano-11,12,13,13a-tetrahydro-9-oxo-9H-imidazo[1,5-a]pyrrolo-[2,1-c][1,4]benzodiazepin-1-carboxylat,

t-Butyl-(S)-11,12,13,13a-tetrahydro-8-(methylthio)-9-oxo-9H-imidazo[1,5-a]-pyrrolo[2,1-c][1,4]benzodiazepin-1-carboxylat,

t-Butyl-(S)-11,12,13,13a-tetrahydro-8-(methylsulfonyl)-9-oxo-9H-imidazo[1,5-a]-pyrrolo[2,1-c][1,4]benzodiazepin-1-carboxylat,

t-Butyl-(S)-7-fluor-11,12,13,13a-tetrahydro-9-oxo-9H-imidazo[1,5-a]pyrrolo-[2,1-c][1,4]benzodiazepin-1-carboxylat und

t-Butyl-(S)-8-chlor-13,13a-dihydro-9-oxo-9H-imidazo[1,5-a]pyrrolo[2,1-c][1,4]-benzodiazepin-1-carboxylat.

22. Verbindungen gemäß einem der Ansprüche 1 bis 21 als pharmazeutische Wirkstoffe.

23. Verbindungen gemäß einem der Ansprüche 1 bis 21 als antikonvulsiv und anxiolytisch wirksame Stoffe.

24. Verfahren zur Herstellung von Verbindungen gemäß einem der Ansprüche 1 bis 21, dadurch gekennzeichnet, daß man

a) eine Verbindung der allgemeinen Formel

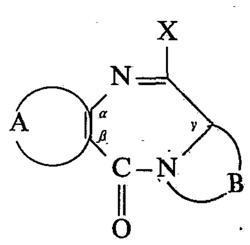

(II)

worin A und B die in Anspruch 1 angegebene Bedeutung besitzen, und X eine Abgangsgruppe bedeutet,
in Gegenwart einer Base mit Isocyanessigsäure-t-butylester umsetzt, oder

b) eine Carbonsäure der allgemeinen Formel

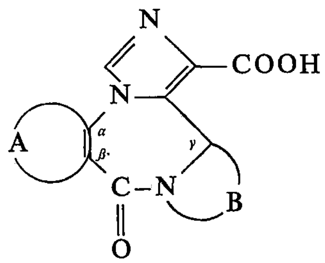

(III)

worin A und B die in Anspruch 1 angegebene Bedeutung besitzen, mit der Maßgabe, daß $R^1$ und/oder $R^2$ nicht Amino bedeuten, falls A die in Anspruch 1 definierte Gruppe (a) bedeutet, in den entsprechenden t-Butylester überführt, oder

c) in einer Verbindung der allgemeinen Formel

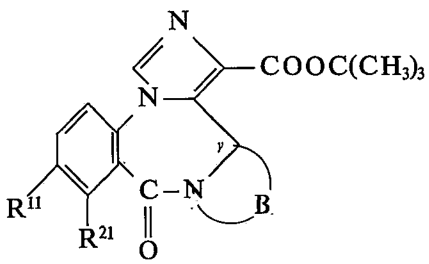

(Ia)

worin B die in Anspruch 1 angegebene Bedeutung besitzt, und entweder $R^{11}$ Halogen und $R^{21}$ Wasserstoff, Trifluormethyl, Amino, Nitro, Cyano oder niederes Alkyl oder $R^{11}$ Wasserstoff, Trifluormethyl, Amino, Nitro, Cyano oder niederes Alkyl und $R^{21}$ Halogen bedeuten, das Halogenatom durch die Cyanogruppe oder, falls $R^{21}$ Halogen bedeutet, auch durch eine niedere Alkylthiogruppe ersetzt, oder

d) in einer Verbindung der allgemeinen Formel

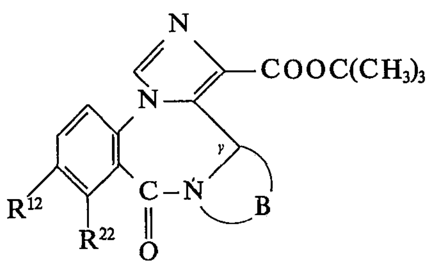

(Ib)

worin B die in Anspruch 1 angegebene Bedeutung besitzt, und entweder $R^{12}$ Amino und $R^{22}$ Wasserstoff, Halogen, Trifluormethyl, Nitro, Cyano oder niederes Alkyl, oder $R^{12}$ Wasserstoff, Halogen, Trifluormethyl, Nitro, Cyano oder niederes Alkyl und $R^{22}$ Amino bedeuten, die Aminogruppe durch ein Wasserstoff- oder Halogenatom oder eine Cyano- oder Nitrogruppe ersetzt, oder

e) eine Verbindung der allgemeinen Formel

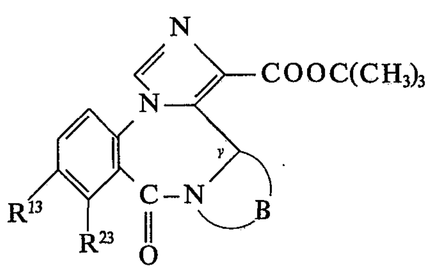

(Ic)

worin B die in Anspruch 1 angegebene Bedeutung besitzt, und einer der Reste $R^{13}$ und $R^{23}$ Amino und der andere Wasserstoff bedeutet, in $\alpha$-Stellung zur Aminogruppe halogeniert, oder

f) in einer Verbindung der allgemeinen Formel

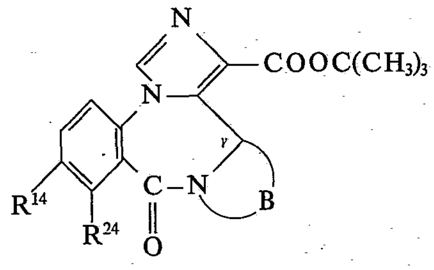

(Id)

worin $R^{14}$ Wasserstoff, Halogen, Trifluormethyl, Nitro, Cyano oder niederes Alkyl und $R^{24}$ niederes Alkylthio oder niederes Alkylsulfinyl bedeuten, und B die in Anspruch 1 angegebene Bedeutung besitzt,
die niedere Alkylthiogruppe zur niederen Alkylsulfinyl- oder Alkylsulfonylgruppe, bzw. die niedere Alkylsulfinylgruppe zur niederen Alkylsulfonylgruppe oxidiert und

g) erwünschtenfalls eine erhaltene Verbindung der allgemeinen Formel I in ein pharmazeutisch annehmbares Säureadditionssalz überführt.

25. Arzneimittel, enthaltend eine Verbindung gemäß einem der Ansprüche 1 bis 21.

26. Antikonvulsiv und anxiolytisch wirksame Mittel, enthaltend eine Verbindung gemäß einem der Ansprüche 1 bis 21.

**Patentansprüche für den Vertragsstaat: AT**

1. Verfahren zur Herstellung von Imidazodiazepinen der allgemeinen Formel

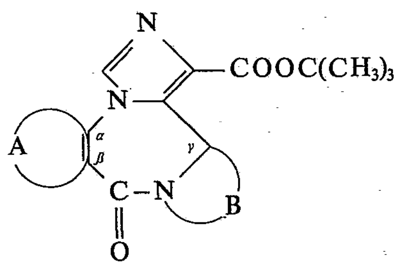

(I)

worin A zusammen mit den beiden mit $\alpha$ und $\beta$ bezeichneten Kohlenstoffatomen die Gruppe

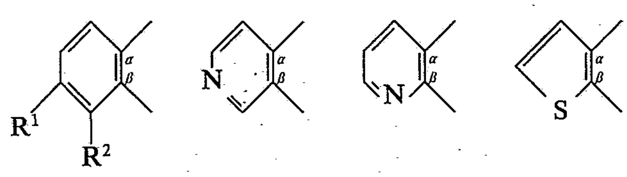

(a) (b) (c) oder (d)

B Dimethylen, Trimethylen oder Propenylen, $R^1$ Wasserstoff, Halogen, Trifluormethyl, Amino, Nitro, Cyano oder niederes Alkyl und $R^2$ Wasserstoff, Halogen, Trifluormethyl, Amino, Nitro, Cyano, niederes Alkyl, niederes Alkyloxy, niederes Alkylthio, niederes Alkylsulfinyl oder niederes Alkylsulfonyl bedeuten, das mit $\gamma$ bezeichnete Kohlenstoffatom die (S)- oder (R,S)-Konfiguration aufweist und die mit nieder bezeichneten Reste höchstens 7 Kohlenstoffatome besitzen,
und pharmazeutisch annehmbaren Säureadditionssalzen davon, dadurch gekennzeichnet, daß man

a) eine Verbindung der allgemeinen Formel

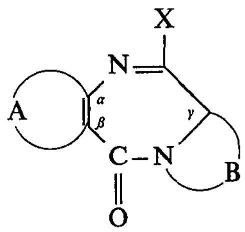

(II)

worin A und B obige Bedeutung besitzen, und X eine Abgangsgruppe bedeutet, in Gegenwart einer Base mit Isocyanessigsäure-t-butylester umsetzt, oder

b) eine Carbonsäure der allgemeinen Formel

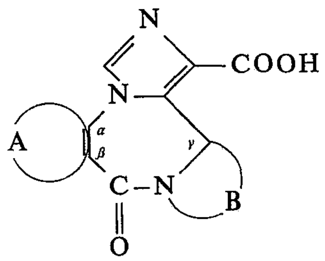

(III)

worin A und B obige Bedeutung besitzen, mit der Maßgabe, daß $R^1$ und/oder $R^2$ nicht Amino bedeuten, falls A die Gruppe (a) bedeutet, in den entsprechenden t-Butylester überführt, oder

c) in einer Verbindung der allgemeinen Formel

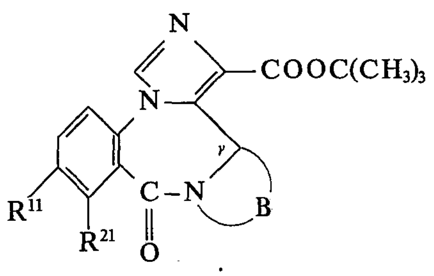

(Ia)

worin B obige Bedeutung besitzt, und entweder $R^{11}$ Halogen und $R^{21}$ Wasserstoff, Trifluormethyl, Amino, Nitro, Cyano oder niederes Alkyl, oder $R^{11}$ Wasserstoff, Trifluormethyl, Amino, Nitro, Cyano oder niederes Alkyl und $R^{21}$ Halogen bedeuten, das Halogenatom durch die Cyanogruppe oder, falls $R^{21}$ Halogen bedeutet, auch durch eine niedere Alkylthiogruppe ersetzt, oder

d) in einer Verbindung der allgemeinen Formel

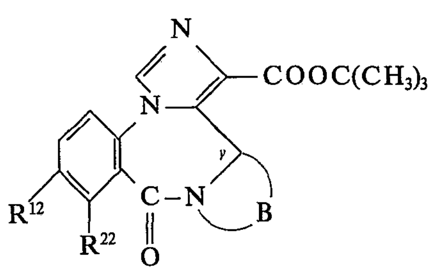

(Ib)

worin B obige Bedeutung besitzt, und entweder $R^{12}$ Amino und $R^{22}$ Wasserstoff, Halogen, Trifluormethyl, Nitro, Cyano oder niederes Alkyl, oder $R^{12}$ Wasserstoff, Halogen, Trifluormethyl, Nitro, Cyano oder niederes Alkyl und $R^{22}$ Amino bedeuten, die Aminogruppe durch ein Wasserstoff- oder Halogenatom oder eine Cyano- oder Nitrogruppe ersetzt, oder

e) eine Verbindung der allgemeinen Formel

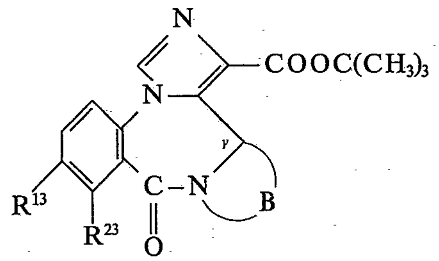

(Ic)

worin B obige Bedeutung besitzt, und einer der Reste $R^{13}$ und $R^{23}$ Amino und der andere Wasserstoff bedeutet,
in $\alpha$-Stellung zur Aminogruppe halogeniert, oder

f) in einer Verbindung der allgemeinen Formel

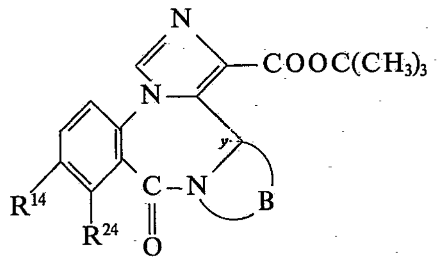

(Id)

worin $R^{14}$ Wasserstoff, Halogen, Trifluormethyl, Nitro, Cyano oder niederes Alkyl und $R^{24}$ niederes Alkylthio oder niederes Alkylsulfinyl bedeuten, und B obige Bedeutung besitzt,
die niedere Alkylthiogruppe zur niederen Alkylsulfinyl- oder Alkylsulfonylgruppe, bzw. die niedere Alkylsulfinylgruppe zur niederen Alkylsulfonylgruppe oxidiert und

g) erwünschtenfalls eine erhaltene Verbindung der allgemeinen Formel I in ein pharmazeutisch annehmbares Säureadditionssalz überführt.

2. Verfahren gemäß Anspruch 1, dadurch gekennzeichnet, daß man Verbindungen der in Anspruch 1 definierten Formel I, worin A die in Anspruch 1 definierte Gruppe (a), einer der Reste $R^1$ und $R^2$ Wasserstoff und der andere Nitro oder Cyano und B Dimethylen, Trimethylen oder Propenylen bedeuten, und das mit $\gamma$ bezeichnete Kohlenstoffatom die (S)- oder (R,S)-Konfiguration aufweist, nach den in Anspruch 1 definierten Verfahren a), b) oder c) herstellt.

3. Verfahren gemäß Anspruch 1, dadurch gekennzeichnet, daß man Verbindungen der in Anspruch 1 definierten Formel I, worin A die in Anspruch 1 definierte Gruppe (a) oder (d), B Dimethylen, $R^1$ Wasserstoff, Halogen oder Trifluormethyl und $R^2$ Wasserstoff, Halogen, Trifluormethyl oder niederes Alkyl bedeuten, und das mit $\gamma$ bezeichnete Kohlenstoffatom die (S)- oder (R,S)-Konfiguration aufweist, und pharmazeutisch annehmbare Säureadditionssalze davon nach den in Anspruch 1 definierten Verfahren a), b) oder g) herstellt.

4. Verfahren gemäß Anspruch 1, dadurch gekennzeichnet, daß man Verbindungen der in Anspruch 1 definierten Formel I, worin A die in Anspruch 1 definierte Gruppe (a) oder (d), B Trimethylen oder Propenylen, $R^1$ Wasserstoff, Halogen oder Trifluormethyl und $R^2$ Wasserstoff, Halogen, Trifluormethyl oder niederes Alkyl bedeuten, und das mit $\gamma$ bezeichnete Kohlenstoffatom die (S)- oder (R,S)-Konfiguration aufweist nach dem in Anspruch 1 definierten Verfahren a) herstellt.

5. Verfahren gemäß Anspruch 1, dadurch gekennzeichnet, daß man Verbindungen der in Anspruch 1 definierten Formel I, worin A die in Anspruch 1 definierte Gruppe (b) oder (c) und B Dimethylen, Trimethylen oder Propenylen bedeuten, und das mit $\gamma$ bezeichnete Kohlenstoffatom die (S)- oder (R,S)-Konfiguration aufweist, und pharmazeutisch annehmbare Säureadditionssalze davon nach den in Anspruch 1 definierten Verfahren a), b) oder g) herstellt.

6. Verfahren gemäß Anspruch 1, dadurch gekennzeichnet, daß man Verbindungen der in Anspruch 1 definierten Formel I, worin A die in Anspruch 1 definierte Gruppe (a), B Dimethylen, Trimethylen oder Propenylen, und entweder $R^1$ niederes Alkyl und $R^2$ Wasserstoff, Halogen, Trifluormethyl, Nitro, Cyano oder niederes Alkyl, oder einer der Reste $R^1$ und $R^2$ Nitro oder Cyano und der andere Halogen, Trifluormethyl, Nitro, Cyano oder niederes Alkyl bedeuten, und das mit $\gamma$ bezeichnete Kohlenstoffatom die (S)- oder (R,S)-Konfiguration aufweist, und pharmazeutisch annehmbare Säureadditionssalze davon nach den in Anspruch 1 definierten Verfahren a), b), c) oder g) herstellt.

7. Verfahren gemäß Anspruch 1, dadurch gekennzeichnet, daß man Verbindungen der in Anspruch 1 definierten Formel I, worin A die in Anspruch 1 definierte Gruppe (a) oder (d), B Trimethylen oder Propenylen, $R^1$ Wasserstoff, Halogen oder Trifluormethylen und $R^2$ Wasserstoff, Halogen, Trifluormethyl oder niederes Alkyl bedeuten, und das mit $\gamma$ bezeichnete Kohlenstoffatom die (S)- oder (R,S)-Konfiguration aufweist, und pharmazeutisch annehmbare Säureadditionssalze davon nach den in Anspruch 1 definierten Verfahren b) oder g) herstellt.

8. Verfahren gemäß Anspruch 1, dadurch gekennzeichnet, daß man Verbindungen der in Anspruch 1 definierten Formel I herstellt, worin A die in Anspruch 1 definierte Gruppe (a), (b), (c) oder (d), $R^1$ Wasserstoff, Amino oder Halogen und $R^2$ Wasserstoff, Halogen, Trifluormethyl, niederes Alkyl, Cyano, Nitro, Amino, niederes Alkyloxy, niederes Alkylthio, niederes Alkylsulfinyl oder niederes Alkylsulfonyl bedeuten.

9. Verfahren gemäß Anspruch 8, dadurch gekennzeichnet, daß man Verbindungen der in Anspruch 1 definierten Formel I herstellt, worin A die in Anspruch 1 definierte Gruppe (a), $R^1$ Wasserstoff oder Halogen und $R^2$ Halogen, Trifluormethyl oder niederes Alkyl bedeuten.

10. Verfahren gemäß einem der Ansprüche 1, 8 und 9, dadurch gekennzeichnet, daß man Verbindungen der in Anspruch 1 definierten Formel I herstellt, worin B Dimethylen oder Trimethylen bedeutet.

11. Verfahren gemäß einem der Ansprüche 1 und 8 bis 10, dadurch gekennzeichnet, daß man eine Verbindung der in Anspruch 1 definierten Formel I herstellt, worin das mit $\gamma$ bezeichnete Kohlenstoffatom die (S)-Konfiguration aufweist.

12. Verfahren gemäß Anspruch 1, dadurch gekennzeichnet, daß man t-Butyl-(S)-12,12a-dihydro-9-oxo-8-(trifluormethyl)-9H,11H-azeto[2,1-c]imidazo[1,5-a][1,4]benzodiazepin-1-carboxylat herstellt.

13. Verfahren gemäß Anspruch 1, dadurch gekennzeichnet, daß man t-Butyl-(S)-12,12a-dihydro-8-methyl-9-oxo-9H,11H-azeto[2,1-c]imidazo[1,5-a][1,4]benzodiazepin-1-carboxylat herstellt.

14. Verfahren gemäß Anspruch 1, dadurch gekennzeichnet, daß man t-Butyl-(S)-8-chlor-7-fluor-11,12,13,13a-tetrahydro-9-oxo-9H-imidazo[1,5-a]pyrrolo[2,1-c][1,4]-benzodiazepin-1-carboxylat herstellt.

15. Verfahren gemäß Anspruch 1, dadurch gekennzeichnet, daß man t-Butyl-(S)-8-chlor-11,12,13,13a-tetrahydro-9-oxo-9H-imidazo[1,5-a]pyrrolo[2,1-c][1,4]benzodiazepin-1-carboxylat herstellt.

16. Verfahren gemäß Anspruch 1, dadurch gekennzeichnet, daß man t-Butyl-(S)-8-äthyl-11,12,13,13a-tetrahydro-9-oxo-9H-imidazo[1,5-a]pyrrolo[2,1-c][1,4]benzodiazepin-1-carboxylat herstellt.

17. Verfahren gemäß Anspruch 1, dadurch gekennzeichnet, daß man t-Butyl-(S)-11,12,13,13a-tetrahydro-9-oxo-8-(trifluormethyl)-9H-imidazo[1,5-a]pyrrolo[2,1-c][1,4]benzodiazepin-1-carboxylat herstellt.

18. Verfahren gemäß Anspruch 1, dadurch gekennzeichnet, daß man t-Butyl-(S)-8-brom-11,12,13,13a-tetrahydro-9-oxo-9H-imidazo[1,5-a]pyrrolo[2,1-c][1,4]benzodiazepin-1-carboxylat herstellt.

19. Verfahren gemäß Anspruch 1, dadurch gekennzeichnet, daß man t-Butyl-(S)-11,12,13,13a-tetrahydro-8-jod-9-oxo-9H-imidazo[1,5-a]pyrrolo[2,1-c][1,4]benzodiazepin-1-carboxylat herstellt.

20. Verfahren gemäß Anspruch 1, dadurch gekennzeichnet, daß man t-Butyl-(S)-8-chlor-12,12a-dihydro-9-oxo-9H,11H-azeto[2,1-c]imidazo[1,5-a][1,4]benzodiazepin-1-carboxylat herstellt.

21. Verfahren gemäß Anspruch 1, dadurch gekennzeichnet, daß man t-Butyl-(S)-11,12,13,13a-tetrahydro-8-methyl-9-oxo-9H-imidazo[1,5-a]pyrrolo[2,1-c][1,4]benzodiazepin-1-carboxylat herstellt.

**Claims for the Contracting States: BE, CH, DE, FR, GB, IT, LI, LU, NL, SE**

1. Imidazodiazepines of the general formula

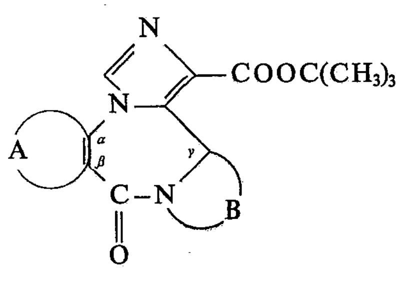

(I)

wherein A together with the two carbon atoms denoted as $\alpha$ and $\beta$ signifies the group

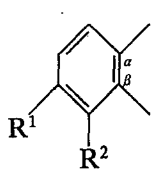 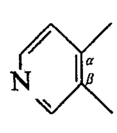 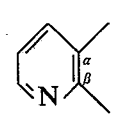 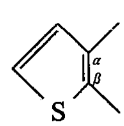

(a) (b) (c) or (d)

B signifies dimethylene, trimethylene or propenylene, $R^1$ signifies hydrogen, halogen, trifluoromethyl, amino, nitro, cyano or lower alkyl and $R^2$ signifies hydrogen, halogen, trifluoromethyl, amino, nitro, cyano, lower alkyl, lower alkyloxy, lower alkylthio, lower alkylsulphinyl or lower alkylsulphonyl, the carbon atom denoted as $\gamma$ has the (S)- or (R,S)-configuration and the residues denoted as lower have a maximum of 7 carbon atoms,
and pharmaceutically acceptable acid addition salts thereof.

2. Compounds in accordance with claim 1, characterized in that A signifies the group (a) defined in claim 1, one of the residues $R^1$ and $R^2$ signifies hydrogen and the other signifies nitro or cyano and B signifies dimethylene, trimethylene or propenylene, and the carbon atom denoted as $\gamma$ has the (S)- or (R,S)-configuration.

3. Compounds in accordance with claim 1, characterized in that A signifies the group (a) or (d) defined in claim 1, B signifies dimethylene, $R^1$ signifies hydrogen, halogen or trifluoromethyl and $R^2$ signifies hydrogen, halogen, trifluoromethyl or lower alkyl, and the carbon atom denoted as $\gamma$ has the (S)- or (R,S)-configuration.

4. Compounds in accordance with claim 1, characterized in that A signifies the group (a) or (d) defined in claim 1, B signifies trimethylene or propenylene, $R^1$ signifies hydrogen, halogen or trifluoromethyl and $R^2$ signifies hydrogen, halogen, trifluoromethyl or lower alkyl, and the carbon atom denoted as $\gamma$ hat the (S)- or (R,S)-configuration.

5. Compounds in accordance with claim 1, characterized in that A signifies the group (b) or (c) defined in claim 1 and B signifies dimethylene, trimethylene or propenylene, and the carbon atom denoted as $\gamma$ has the (S)- or (R,S)-configuration.

6. Compounds in accordance with claim 1, characterized in that A signifies the group (a) defined in claim 1, B signifies dimethylene, trimethylene or propenylene, and either $R^1$ signifies lower alkyl and $R^2$ signifies hydrogen, halogen, trifluoromethyl, nitro, cyano or lower alkyl or one of the residues $R^1$ and $R^2$ signifies nitro or cyano and the other signifies halogen, trifluoromethyl, nitro, cyano or lower alkyl, and the carbon atom denoted as $\gamma$ has the (S)- or (R,S)-configuration.

7. Compounds in accordance with claim 1, characterized in that A signifies the group (a), (b), (c) or (d) defined in claim 1, $R^1$ signifies hydrogen, amino or halogen and $R^2$ signifies hydrogen, halogen, trifluoromethyl, lower alkyl, cyano, nitro, amino, lower alkyloxy, lower alkylthio, lower alkylsulphinyl or lower alkylsulphonyl.

8. Compounds in accordance with claim 7, characterized in that $R^1$ signifies hydrogen or halogen and $R^2$ signifies halogen, trifluoromethyl or lower alkyl.

9. Compounds in accordance with any one of claims 1, 7 and 9, characterized in that B signifies dimethylene or trimethylene.

10. Compounds in accordance with any one of claims 1 and 7 to 9, characterized in that the carbon atom denoted as $\gamma$ has the (S)-configuration.

11. t-Butyl (S)-12,12a-dihydro-9-oxo-8-(trifluoromethyl)-9H,11H-azeto[2,1-c]imidazo[1,5-a][1,4]benzodiazepine-1-carboxylate.

12. t-Butyl (S)-12,12a-dihydro-8-methyl-9-oxo-9H,11H-azeto[2,1-c]imidazo[1,5-a][1,4]benzodiazepine-1-carboxylate.

13. t-Butyl (S)-8-chloro-7-fluoro-11,12,13,13a-tetrahydro-9-oxo-9H-imidazo[1,5-a]pyrrolo[2,1-c][1,4]benzodiazepine-1-carboxylate.

14. t-Butyl (S)-8-chloro-11,12,13,13a-tetrahydro-9-oxo-9H-imidazo[1,5-a]pyrrolo[2,1-c][1,4]benzodiazepine-1-carboxylate.

15. t-Butyl (S)-8-ethyl-11,12,13,13a-tetrahydro-9-oxo-9H-imidazo[1,5-a]pyrrolo[2,1-c][1,4]benzodiazepine-1-carboxylate.

16. t-Butyl (S)-11,12,13,13a-tetrahydro-9-oxo-8-(trifluoromethyl)-9H-imidazo[1,5-a]pyrrolo[2,1-c][1,4]benzodiazepine-1-carboxylate.

17. t-Butyl (S)-8-bromo-11,12,13,13a-tetrahydro-9-oxo-9H-imidazo[1,5-a]pyrrolo[2,1-c][1,4]benzodiazepine-1-carboxylate.

18. t-Butyl (S)-11,12,13,13a-tetrahydro-8-iodo-9-oxo-9H-imidazo[1,5-a]pyrrolo[2,1-c][1,4]benzodiazepine-1-carboxylate.

19. t-Butyl (S)-8-chloro-12,12a-dihydro-9-oxo-9H,11H-azeto[2,1-c]imidazo[1,5-a][1,4]benzodiazepine-1-carboxylate.

20. t-Butyl (S)-11,12,13,13a-tetrahydro-8-methyl-9-oxo-9H-imidazo[1,5-a]pyrrolo[2,1-c][1,4]benzodiazepine-1-carboxylate.

21. t-Butyl 8-chloro-11,13a-dihydro-9-oxo-9H-imidazo[1,5-a]pyrrolo[2,1-c][1,4]benzodiazepine-1-carboxylate,

t-butyl-(S)-10,11,12,12a-tetrahydro-8-oxo-8H-imidazo[5,1-c]pyrrolo[1,2-a]-thieno[3,2-e]-[1,4]diazepine-1-carboxylate,

t-butyl (S)-11,12,13,13a-tetrahydro-9-oxo-9H-imidazo[1,5-a]pyrrolo[2,1-c][1,4]-benzodiazepine-1-carboxylate,

t-butyl (S)-7-chloro-11,12,13,13a-tetrahydro-9-oxo-9H-imidazo[1,5-a]pyrrolo-[2,1-c][1,4]benzodiazepine-1-carboxylate,

t-butyl (S)-8-amino-11,12,13,13a-tetrahydro-9-oxo-9H-imidazo[1,5-a]pyrrolo-

[2,1-c][1,4]benzodiazepine-1-carboxylate,
t-butyl (S)-11,12,13,13a-tetrahydro-8-nitro-9-oxo-9H-imidazo[1,5-a]pyrrolo-[2,1-c][1,4]benzodiazepine-1-carboxylate,
t-butyl (S)-8-cyano-11,12,13,13a-tetrahydro-9-oxo-9H-imidazo[1,5-a]pyrrolo-[2,1-c][1,4]benzodiazepine-1-carboxylate,
t-butyl (S)-11,12,13,13a-tetrahydro-8-(methylthio)-9-oxo-9H-imidazo[1,5-a]-pyrrolo[2,1-c][1,4]benzodiazepine-1-carboxylate,
t-butyl (S)-11,12,13,13a-tetrahydro-8-(methylsulphonyl)-9-oxo-9H-imidazo-[1,5-a]pyrrolo[2,1-c][1,4]benzodiazepine-1-carboxylate,
t-butyl (S)-7-fluoro-11,12,13,13a-tetrahydro-9-oxo-9H-imidazo[1,5-a]pyrrolo-[2,1-c][1,4]benzodiazepine-1-carboxylate and
t-butyl (S)-8-chloro-13,13a-dihydro-9-oxo-9H-imidazo[1,5-a]pyrrolo-[2,1-c][1,4]benzodiazepine-1-carboxylate.

22. Compounds in accordance with any one of claims 1 to 21 as pharmaceutically active substances.

23. Compounds in accordance with any one of claims 1 to 21 as anticonvulsive and anxiolytic active substances.

24. A process for the manufacture of compounds in accordance with any one of claims 1 to 21, characterized by

a) reacting a compound of the general formula

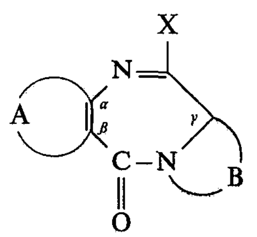

(II)

wherein A and B have the significance given in claim 1 and X signifies a leaving group, in the presence of a base with isocyanoacetic acid t-butyl ester, or

b) converting a carboxylic acid of the general formula

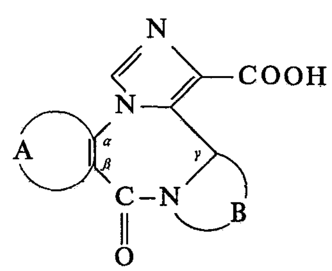

(III)

wherein A and B habe the significance given in claim 1, with the proviso that $R^1$ and/or $R^2$ does not signify amino when A signifies the group (a) defined in claim 1, into the corresponding t-butyl ester, or

c) replacing the halogen atom in a compound of the general formula

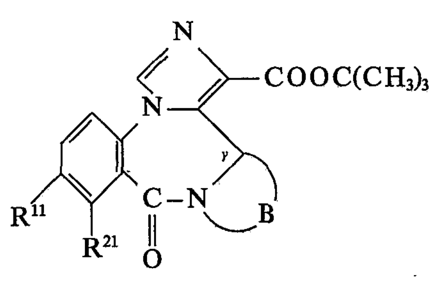

(Ia)

wherein B has the significance given in claim 1 and either $R^{11}$ signifies halogen and $R^{21}$ signifies hydrogen, trifluoromethyl, amino, nitro, cyano or lower alkyl or $R^{11}$ signifies hydrogen, trifluoromethyl, amino, nitro, cyano or lower alkyl and $R^{21}$ signifies halogen, by the cyano group or, where $R^{21}$ signifies halogen, also by a lower alkylthio group, or

d) replacing the amino group in a compound of the general formula

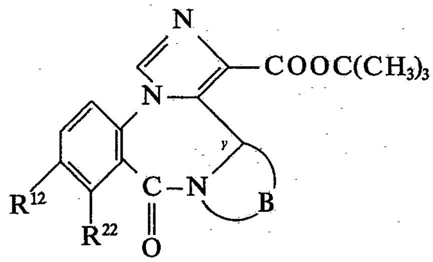

(Ib)

wherein B has the significance given in claim 1 and either $R^{12}$ signifies amino and $R^{22}$ signifies hydrogen, halogen, trifluoromethyl, nitro, cyano or lower alkyl or $R^{12}$ signifies hydrogen, halogen, trifluoromethyl, nitro, cyano or lower alkyl and $R^{22}$ signifies amino,
by a hydrogen or halogen atom or a cyano or nitro group, or

e) halogenating a compound of the general formula

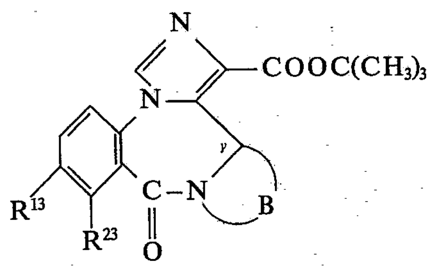

(Ic)

wherein B has the significance given in claim 1 and one of the residues $R^{13}$ and $R^{23}$ signifies amino and the other signifies hydrogen.
in the $\alpha$-position to the amino group, or

f) oxidizing the lower alkylthio group to the lower alkylsulphinyl or alkylsulphonyl group or the lower alkylsulphinyl group to the lower alkylsulphonyl group in a compound of the general formula

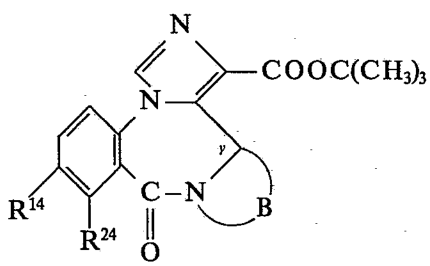

(Id)

wherein $R^{14}$ signifies hydrogen, halogen, trifluoromethyl, nitro, cyano or lower alkyl and $R^{24}$ signifies lower alkylthio or lower alkylsulphinyl, and B has the significance given in claim 1,
and

g) if desired, converting a compound of general formula I obtained into a pharmaceutically acceptable acid addition salt.

25. Medicaments containing a compound in accordance with any one of claims 1 to 21.

26. Anticonvulsive or anxiolytic medicaments containing a compound in accordance with any one of claims 1 to 21.

**Claims for the Contracting State: AT**

1. A process for the manufacture of imidazodiazepines of the general formula

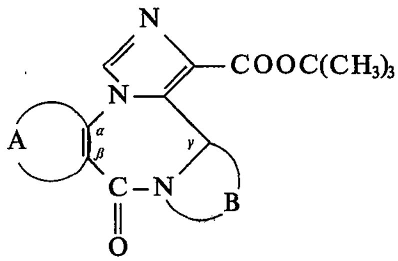

(I)

wherein A together with the two carbon atoms denoted as $\alpha$ and $\beta$ signifies the group

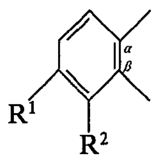 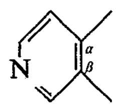 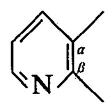 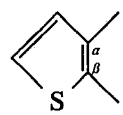

(a) (b) (c) or (d)

B signifies dimethylene, trimethylene or propenylene, $R^1$ signifies hydrogen, halogen, trifluoromethyl, amino, nitro, cyano or lower alkyl and $R^2$ signifies hydrogen, halogen, trifluoromethyl, amino, nitro, cyano, lower alkyl, lower alkyloxy, lower alkylthio, lower alkylsulphinyl or lower alkylsulphonyl, the carbon atom denoted as $\gamma$ has the (S)- or (R,S)-configuration and the residues denoted as lower have a maximum of 7 carbon atoms,
and pharmaceutically acceptable acid addition salts thereof, characterized by

a) reacting a compound of the general formula

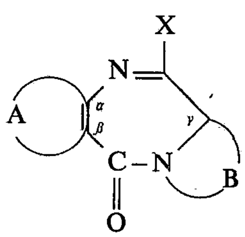

(II)

wherein A and B have the above significance and X signifies a leaving group, in the presence of a base with isocyanoacetic acid t-butylester, or

b) converting a carboxylic acid of the general formula

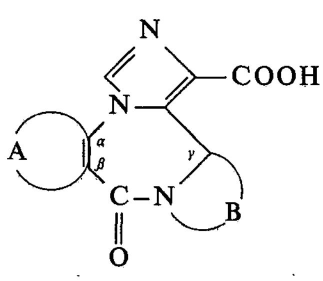

(III)

wherein A and B have the above significance, with the proviso that $R^1$ and/or $R^2$ does not signify amino when A signifies the group (a),
into the corresponding t-butyl ester, or

c) replacing the halogen atom in a compound of the general formula

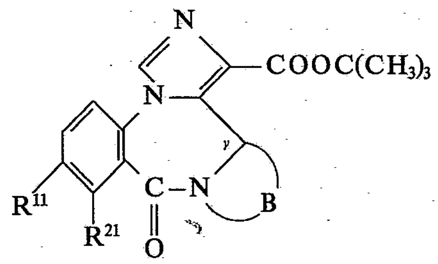

(Ia)

wherein B has the above significance and either $R^{11}$ signifies halogen and $R^{21}$ signifies hydrogen, trifluoromethyl, amino, nitro, cyano or lower alkyl or $R^{11}$ signifies hydrogen, trifluoromethyl, amino, nitro, cyano or lower alkyl and $R^{21}$ signifies halogen,
by the cyano group or, where $R^{21}$ signifies halogen, also by a lower alkylthio group, or

d) replacing the amino group in a compound of the general formula

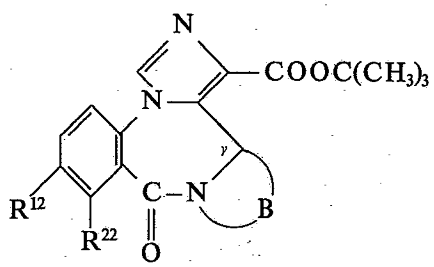

(Ib)

wherein B has the above significance and either $R^{12}$ signifies amino and $R^{22}$ signifies hydrogen, halogen, trifluoromethyl, nitro, cyano or lower alkyl or $R^{12}$ signifies hydrogen, halogen, trifluoromethyl, nitro, cyano or lower alkyl and $R^{22}$ signifies amino,
by a hydrogenatom or a cyano or nitro group, or

e) halogenating a compound of the general formula

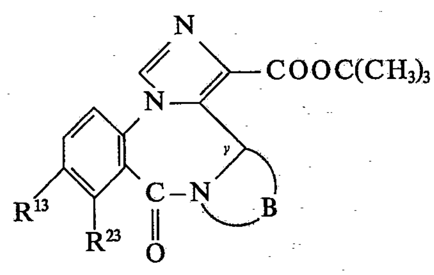

(Ic)

wherein B has the above significance and one of the residues $R^{13}$ and $R^{23}$ signifies amino and the other signifies hydrogen,
in the $\alpha$-position to the amino group, or

f) oxidizing the lower alkylthio group to the lower alkylsulphinyl or alkylsulphonyl group or the lower alkylsulphinyl group to the lower alkylsulphonyl group in a compound of the general formula

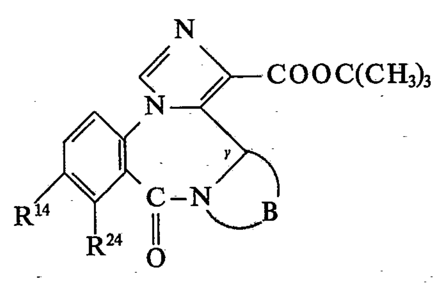

(Id)

wherein $R^{14}$ signifies hydrogen, halogen, trifluoromethyl, nitro, cyano or lower alkyl and $R^{24}$ signifies lower alkylthio or lower alkylsulphinyl, and B has the above significance,
and

g) if desired, converting a compound of the general formula I obtained into a pharmaceutically aceptable acid addition salt.

2. A process in accordance with claim 1, characterized in that compounds of formula I defined in claim 1 in which A signifies the group (a) defined in claim 1, one of the residues $R^1$ and $^2$ signifies hydrogen and the other signifies nitro or cyano and B signifies dimethylene, trimethylene or propenylene, and the carbon atom denoted as $\gamma$ has the (S)- or (R,S)-configuration, are manufactured according to process a), b) or c) defined in claim 1.

3. A process in accordance with claim 1, characterized in that compounds of formula I defined in claim 1 in which A signifies the group a) or d) defined in claim 1, B signifies dimethylene, $R^1$ signifies hydrogen, halogen or trifluoromethyl and $R^2$ signifies hydrogen, halogen, trifluoromethyl or lower alkyl, and the carbon atom denoted as $\gamma$ has the (S)- or (R,S)-configuration, and pharmaceutically acceptable acid addition salts thereof are manufactured according to process a), b) or g) defined in claim 1.

4. A process in accordance with claim 1, characterized in that compounds of formula I defined in claim 1 in which A signifies the group (a) or (d) defined in claim 1, B signifies trimethylene or propenylene $R^1$ signifies hydrogen, halogen or trifluoromethyl and $R^2$ signifies hydrogen, halogen, trifluoromethyl or lower alkyl, and the carbonatom denoted as $\gamma$ has the (S)- or (R,S)-configuration, are manufactured according to process a) defined in claim 1.

5. A process in accordance with claim 1, characterized in the compounds of formula I defined in claim 1 in which A signifies the group (b) or (c) defined in claim 1 and B signifies dimethylene, trimethylene or propenylene, and the carbon atom denoted as $\gamma$ has the (S)- or (R,S)-configuration, and pharmaceutically acceptable acid addition salts thereof are manufactured according to process a), b) or g) defined in claim 1.

6. A process in accordance with claim 1, characterized in that compounds of formula 1 defined in claim 1 in which A signifies the group (a) defined in claim 1, B signifies dimethylene, trimethylene or propenylene, and either $R^1$ signifies lower alkyl and $R^2$ signifies hydrogen, halogen, trifluoromethyl, nitro, cyano or lower alkyl, or one of the residues $R^1$ and $R^2$ signifies nitro or cyano and the other signifies halogen, trifluoromethyl, nitro, cyano or lower alkyl, and the carbon atom denoted as $\gamma$ has the (S)- or (R,S)-configuration, and pharmaceutically acceptable acid addition salts thereof are manufactured according to process a), b), c) or g) defined in claim 1.

7. A process in accordance with claim 1, characterized in that compounds of formula I defined in claim 1 in which A signifies the group (a) or (d) defined in claim 1, B signifies trimethylene or propenylene, $R^1$ signifies hydrogen, halogen or trifluoromethyl and $R^2$ signifies, hydrogen, halogen, trifluoromethyl or lower alkyl, and the carbon atom denoted as $\gamma$ has the (S)- or (R,S)-configuration, and pharmaceutically acceptable acid addition salts thereof are manufactured according to process b) or g) defined in claim 1.

8. A process in accordance with claim 1, characterized in that compounds of formula I defined in claim 1 in which A signifies the group (a), (b), (c) or (d) defined in claim 1, $R^1$ signifies hydrogen, amino or halogen and $R^2$ signifies hydrogen, halogen, trifluoromethyl, lower alkyl, cyano, nitro, amino, lower alkyloxy, lower alkylthio, lower alkylsulphinyl or lower alkylsulphonyl are manufactured.

9. A process in accordance with claim 8, characterized in that compounds of formula I defined in claim 1 in which a signifies the group (a) in claim 1, $R^1$ signifies hydrogen or halogen and $R^2$ signifies halogen, trifluoromethyl or lower alkyl are manufactured.

10. A process in accordance with any one of claims 1, 8 and 9, characterized in that compounds of formula I defined in claim 1 in which B signifies dimethylene or trimethylene are manufactured.

11. A process in accordance with any one of claims 1 and 8 to 10, characterized in that a compound of formula I defined in claim 1 in which the carbon atom denoted as $\gamma$ has the (S)-configuration is manufactured.

12. A process in accordance with claim 1, characterized in that t-butyl (S)-12,12a-dihydro-9-oxo-8-(trifluoromethyl)-9H,11H-azeto[2,1-c]imidazo[1,5-a][1,4]benzodiazepine-1-carboxylate is manufactured.

13. A process in accordance with claim 1, characterized in that t-butyl (S)-12,12a-dihydro-8-methyl-9-oxo-9H,11H-azeto[2,1-c]imidazo[1,5-a][1,4]benzodiazepine-1-carboxylate is manufactured.

14. A process in accordance with claim 1, characterized in that t-butyl (S)-8-chloro-7-fluoro-11,12,13,13a-tetrahydro-9-oxo-9H-imidazo[1,5-a]pyrrolo[2,1-c][1,4]benzodiazepine-1-carboxylate is manufactured.

15. A process in accordance with claim 1, characterized in that t-butyl (S)-8-chloro-11,12,13,13a-tetrahydro-9-oxo-9H-imidazo[1,5-a]pyrrolo[2,1-c][1,4]benzodiazepine-1-carboxylate is manufactured.

16. A process in accordance with claim 1, characterized in that t-butyl (S)-8-ethyl-11,12,13,13a-tetrahydro-9-oxo-9H-imidazo[1,5-a]pyrrolo[2,1-c][1,4]benzodiazepine-1-carboxylate is manufactured.

17. A process in accordance with claim 1, characterized in that t-butyl (S)-11,12,13,13a-tetrahydro-9-oxo-8-(trifluoromethyl)-9H-imidazo[1,5-a]pyrrolo[2,1-c][1,4]benzodiazepine-1-carboxylate is manufactured.

18. A process in accordance with claim 1, characterized in that t-butyl (S)-8-bromo-11,12,13,13a-tetrahydro-9-oxo-9H-imidazo[1,5-a]pyrrolo[2,1-c][1,4]benzodiazepine-1-carboxylate is manufactured.

19. A process in accordance with claim 1, characterized in that t-butyl (S)-11,12,13,13a-tetrahydro-8-iodo-9-oxo-9H-imidazo[1,5-a]pyrrolo[2,1-c][1,4]benzodiazepine-1-carboxylate is manufactured.

20. A process in accordance with claim 1, characterized in that t-butyl (S)-8-chloro-12,12a-dihydro-9-oxo-9H,11H-azeto[2,1-c]imidazo[1,5-a][1,4]benzodiazepine-1-carboxylate is manufactured.

21. A process in accordance with claim 1, characterized in that t-butyl (S)-11,12,13,13a-tetrahydro-8-methyl-9-oxo-9H-imidazo[1,5-a]pyrrolo[2,1-c][1,4]benzodiazepine-1-carboxylate is manufactured.

**Revendications pour les états contractants: BE, CH, DE, FR, GB, IT, LI, LU, NL, SE**

1. Imidazodiazépines de formule générale

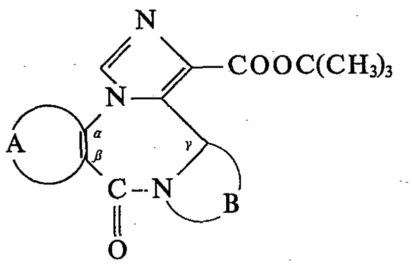

(I)

dans laquelle A représente avec les deux atomes de carbone marqués $\alpha$ et $\beta$ le groupe

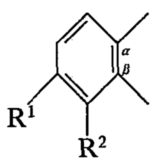 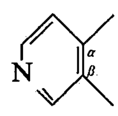 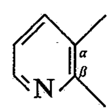 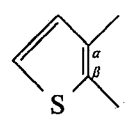

(a) (b) (c) ou (d)

B représente un groupe diméthylène, triméthylène ou propénylèn, $R^1$ représente l'hydrogène, un halogène,un groupe trifluorométhyle, amino, nitro, cyano ou alkyle inférieur et $R^2$ représente l'hydrogène, un halogène, un groupe trifluorométhyle, amino, nitro, cyano, alkyle inférieur, alkyloxy inférieur, alkylthio inférieur, alkylsulfinyle inférieur ou alkylsulfonyle inférieur, l'atome de carbone marqué $\gamma$ présente la configuration (S) ou (R,S), et dont les groupes dits inférieurs possèdent au plus 7 atomes de carbone et leurs sels formés par addition avec des acides acceptables pour l'usage pharmaceutique.

2. Composés selon la revendication 1, caractérisés en ce que A représente le groupe (a) défini dans la revendication 1, l'un des symboles $R^1$ et $R^2$ représente l'hydrogène et l'autre un groupe nitro ou cyano, et B représente un groupe diméthylène, triméthylène ou propénylène, et l'atomé de carbone marqué $\gamma$ présente la configuration (S) ou (R,S).

3. Composés selon la revendication 1, caractérisés en ce que A représente le groupe (a) ou (d) défini dans la revendication 1, B représente un groupe diméthylène, $R^1$ représente l'hydrogène, un halogène ou un groupe trifluorométhyle et $R^2$ représente l'hydrogène, un halogène, un groupe trifluorométhyle ou alkyle inférieur, et l'atome de carbone marqué $\gamma$ présente la configuration (S) ou (R,S).

4. Composés selon la revendication 1, caractérisés en ce que A représente le groupe (a) ou le groupe (d) défini dans la revendication 1, B représente un groupe triméthylène ou propénylène, $R^1$ représente l'hydrogène, un halogène ou un groupe trifluorométhyle et $R^2$ représente l'hydrogène, un halogène, un groupe trifluorométhyle ou alkyle inférieur, et l'atome de carbone marqué $\gamma$ présente la configuration (S) ou (R,S).

5. Composés selon la revendication 1, caractérisés en ce que A représente le groupe (b) ou (c) défini dans la revendication 1 et B représente un groupe diméthylène, triméthylène ou propénylène, et l'atome de carbone marqué $\gamma$ présente la configuration (S) ou (R,S).

6. Composés selon la revendication 1, caractérisés en ce que A représente le groupe (a) défini dans la revendication 1, B représente un groupe diméthylène, triméthylène ou propénylène, et ou bien $R^1$ représente un groupe alkyle inférieur et $R^2$ l'hydrogène, un halogène, un groupe trifluorométhyle, nitro, cyano ou alkyle inférieur, ou bien l'un des symboles $R^1$ et $R^2$ représente un groupe nitro ou cyano et l'autre un halogène, un groupe trifluorométhyle, nitro, cyano ou alkyle inférieur, et l'atome de carbone marqué $\gamma$ présente la configuration (S) ou (R,S).

7. Composés selon la revendication 1, caractérisés en ce que A représente le groupe (a), (b), (c) ou (d) défini dans la revendication 1, $R^1$ représente l'hydrogène, un groupe amino ou un halogène et $R^2$ représente l'hydrogène, un halogène, un groupe trifluorométhyle, alkyle inférieur, cyano, nitro, amino, alkyloxy inférieur, alkylthio inférieur, alkylsulfinyle inférieur ou alkylsulfonyle inférieur.

8. Composés selon la revendication 7, caractérisés en ce que $R^1$ représente l'hydrogène ou un halogène et $R^2$ représente un halogène, un groupe trifluorométhyle ou alkyle inférieur.

9. Composés selon l'une des revendications 1, 7 et 8, caractérisés en ce que B représente le groupe diméthylène ou triméthylène.

10. Composés selon l'une des revendication 1 et 7 à 9, caractérisés en ce que l'atome de carbone marqué $\gamma$ présente la configuration (S).

11. Le (S)-12,12a-dihydro-9-oxo-8-(trifluorométhyl)-9H,11H-azéto[2,1-c]imidazo[1,5-a][1,4]benzodiazépine-1-carboxylate de tert.-butyle.

12. Le (S)-12,12a-dihydro-8-méthyl-9-oxo-9H,11H-azéto[2,1-c]imidazo[1,5-a][1,4]benzodiazépine-1-carboxylate de tert.-butyle.

13. Le (S)-8-chloro-7-fluoro-11,12,13,13a-tétrahydro-9-oxo-9H-imidazo[1,5-a]pyrrolo[2,1-c][1,4]benzodiazépine-1-carboxylate de tert.-butyle.

14. Le (S)-8-chloro-11,12,13,13a-tétrahydro-9-oxo-9H-imidazo[1,5-a]pyrrolo[2,1-c][1,4]benzodiazépine-1-carboxylate de tert.-butyle.

15. Le (S)-8-éthyl-11,12,13,13a-tétrahydro-9-oxo-9H-imidazo[1,5-a]pyrrolo[2,1-c][1,4]benzodiazépine-1-carboxylate de tert.-butyle.

16. Le (S)-11,12,13,13a-tétrahydro-9-oxo-8-(trifluorométhyl)-9H-imidazo[1,5-a]pyrrolo[2,1-c][1,4]benzodiazépine-1-carboxylate de tert.-butyle.

17. Le (S)-8-bromo-11,12,13,13a-tétrahydro-9-oxo-9H-imidazo[1,5-a]pyrrolo[2,1-c][1,4]benzodiazépine-1-carboxylate de tert.-butyle.

18. Le (S)-11,12,13,13a-tétrahydro-8-iodo-9-oxo-9H-imidazo[1,5-a]pyrrolo[2,1-c][1,4]benzodiazépine-1-carboxylate de tert.-butyle.

19. Le (S)-8-chloro-12,12a-dihydro-9-oxo-9H,11H-azéto[2,1-c]imidazo[1,5-a][1,4]benzodiazépine-1-carboxylate de tert.-butyle.

20. Le (S)-11,12,13,13a-tétrahydro-8-méthyl-9-oxo-9H-imidazo[1,5-a]pyrrolo[2,1-c][1,4]benzodiazépine-1-carboxylate de tert.-butyle.

21. Le 8-chloro-11,13a-dihydro-9-oxo-9H-imidazo[1,5-a]pyrrolo[2,1-c][1,4]benzodiazépine-1-carboxylate de tert.-butyle,

Le (S)-10,11,12,12a-tétrahydro-8-oxo-8H-imidazo[5,1-c]pyrrolo[1,2-a]thiéno[3,2-e][1,4]diazépine-1-carboxylate de tert.-butyle,

Le (S)-11,12,13,13a-tétrahydro-9-oxo-9H-imidazo[1,5-a]pyrrolo[2,1-c][1,4]benzodiazépine-1-carboxylate de tert.-butyle,

Le (S)-7-chloro-11,12,13,13a-tétrahydro-9-oxo-9H-imidazo[1,5-a]pyrrolo[2,1-c][1,4]benzodiazépine-1-carboxylate de tert.-butyle,

Le (S)-8-amino-11,12,13,13a-tétrahydro-9-oxo-9H-imidazo[1,5-a]pyrrolo[2,1-c][1,4]benzodiazépine-1-carboxylate de tert.-butyle,

Le (S)-11,12,13,13a-tétrahydro-8-nitro-9-oxo-9H-imidazo[1,5-a]pyrrolo[2,1-c][1,4]benzodiazépine-1-carboxylate de tert.-butyle,

Le (S)-8-cyano-11,12,13,13a-tétrahydro-9-oxo-9H-imidazo[1,5-a]pyrrolo[2,1-c][1,4]benzodiazépine-1-carboxylate de tert.-butyle,

Le (S)-11,12,13,13a-tétrahydro-8-(méthylthio)-9-oxo-9H-imidazo[1,5-a]pyrrolo[2,1-c][1,4]benzodiazépine-1-carboxylate de tert.-butyle,

Le (S)-11,12,13,13a-tétrahydro-8-(méthylsulfonyl)-9-oxo-9H-imidazo[1,5-a]pyrrolo[2,1-c][1,4]benzodiazépine-1-carboxylate de tert.-butyle,

Le (S)-7-fluoro-11,12,13,13a-tétrahydro-9-oxo-9H-imidazo[1,5-a]pyrrolo[2,1-c][1,4]benzodiazépine-1-carboxylate de tert.-butyle,

Le (S)-8-chloro-13,13a-dihydro-9-oxo-9H-imidazo[1,5-a]pyrrolo[2,1-c][1,4]benzodiazépine-1-carboxylate de tert.-butyle.

22. A titre de substances actives pharmaceutiques, les composés selon l'une des revendications 1 à 21.

23. A titre de substances actives anticonvulsives et anxiolytiques les composés selon l'une des revendications 1 à 21.

24. Procédé de préparation des composés selon l'une des revendications 1 à 21, caractérisé en ce que

(a) on fait réagir un composé de formule générale

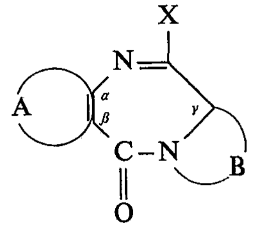

(II)

dans laquelle A et B ont les significations indiquées dans le revendication 1, et X représente une groupe éliminable, en présence d'une base avec l'isocyanacétate de tert.-butyle, ou bien

(b) on convertit un acide carboxylique de formule générale

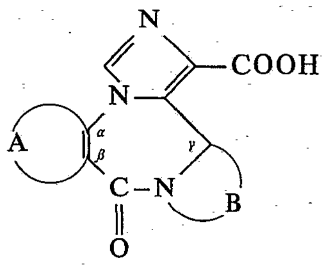

(III)

dans laquelle A et B ont les significations indiquées dans la revendication 1, étant spécifié que $R^1$ et/ou $R^2$ ne peuvent représenter un groupe amino lorsque A représente le groupe (a) défini dans la revendication 1, en l'ester tert.-butylique correspondant, ou bien

(c) dans un composé de formule générale

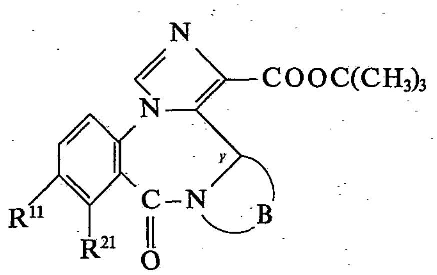

(Ia)

dans laquelle B a la siginification indiquée dans la revendication I, et ou bien $R^{11}$ représente un halogène et $R^{21}$ l'hydrogène, un groupe trifluorométhyle, amino, nitro, cyano ou alkyle inférieur, ou bien $R^{11}$ représente l'hydrogène, un groupe trifluorométhyle, amino, nitro, cyano ou alkyle inférieur et $R^{21}$ un halogène,

on remplace l'atome d'halogène par le groupe cyano ou bien encore, lorsque $R^{21}$ représente un halogène, par un groupe alkylthio inférieur, ou bien

(d) dans un composé de formule générale

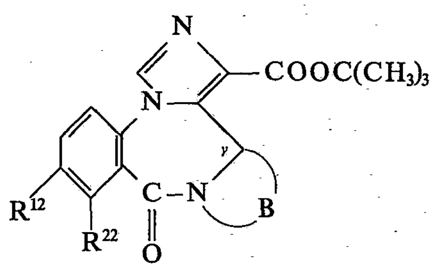

(Ib)

dans laquelle B a la signification indiquée dans la revendication 1, et ou bien $R^{12}$ représente un groupe amino et $R^{22}$ l'hydrogène, un halogène, un groupe trifluorométhyle, nitro, cyano ou alkyle inférieur, ou bien $R^{12}$ représente l'hydrogène, un halogène, un groupe trifluorométhyle, nitro, cyano ou alkyle inférieur et $R^{22}$ représente un groupe amino,

on remplace le groupe amino par un atome l'hydrogène ou d'halogène ou par un groupe cyano ou nitro, ou bien

(e) on halogène un composé de formule générale

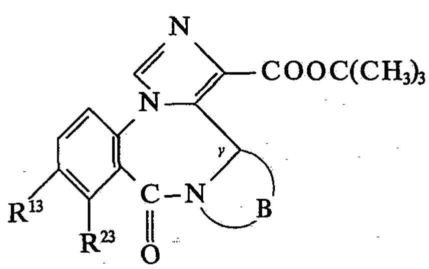

(Ic)

dans laquelle B a la signification indiquée dans la revendication 1, et l'un des symboles $R^{13}$ et $R^{23}$ représente un groupe amino et l'autre l'hydrogène,
en position $\alpha$ du groupe amino, ou bien

(f) dans un composé de formule générale

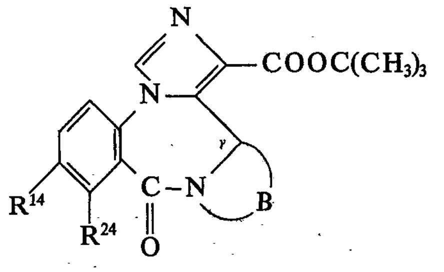

(Id)

dans laquelle $R^{14}$ représente l'hydrogène, un halogène, un groupe trifluorométhyle, nitro, cyano ou alkyle inférieur et $R^{24}$ représente un groupe alkylthio inférieur ou alkylsulfinyle inférieur, B ayant la signification indiquée dans la revendication 1,
on oxyde le groupe alkylthio inférieur en groupe alkylsulfinyle inférieur ou alkylsulfonyle inférieur ou le groupe alkylsulfinyle inférieur en groupe alkylsulfonyle inférieur et

(g) si on le désire, on convertit un composé obtenu de formule générale I en un sel formé par addition avec un acide acceptable pour l'usage pharmaceutique.

25. Médicaments comprenant un composé selon l'une des revendications 1 à 21.

26. Agents actifs anticonvulsifs et anxiolytiques comprenant un composé selon l'une des revendications l à 21.

**Revendications pour l'état contractant: AT**

1. Procédé de préparation d'imidazodiazépines de formule générale

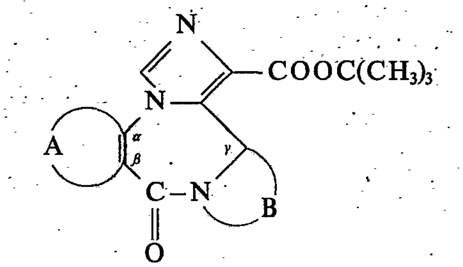

(I)

dans laquelle A représente avec les deux atomes de carbone marqués $\alpha$ et $\beta$ le groupe

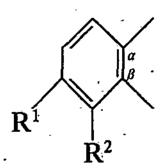 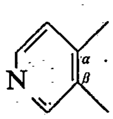 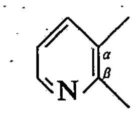 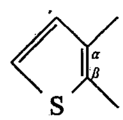

(a) (b) (c) ou (d)

B représente un groupe diméthylène, triméthylène ou propénylèn, $R^1$ représente l'hydrogène, un halogène, un groupe trifluorométhyle, amino, nitro, cyano ou alkyle inférieur et $R^2$ représente l'hydrogène, un halogène, un groupe trifluorométhyle, amine, nitro, cyano, alkyle inférieur, alkyloxy inférieur, alkylthio inférieur, alkylsulfinyle inférieur ou alkylsulfonyle inférieur, l'atome de carbone marqué $\gamma$ présente la configuration (S) ou (R,S), et dont les groupes dits inférieur possèdent au plus 7 atomes de carbone,
et de leurs sels formés par addition avec des acides acceptables pour l'usage pharmaceutique, caractérisé en ce que

a) on fait réagir un composé de formule générale

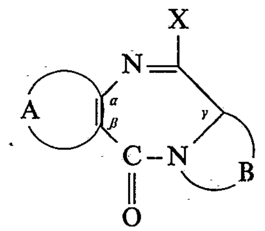

(II)

dans laquelle A et B ont les significations indiquées ci-dessus, et X représente un groupe éliminable,
en présence d'une base avec l'isocyanacétate de tert.-butyle, ou bien

b) on convertit un acide carboxylique de formule générale

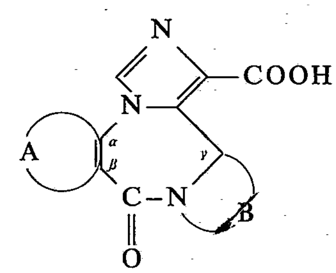

(III)

dans laquelle A et B ont les significations indiquées ci-dessus, étant spécifié que $R^1$ et/ou $R^2$ ne peuvent représenter un groupe amino lorsque A représente le groupe (a),
en l'ester tert.-butylique correspondant, ou bien

c) dans un composé de formule générale

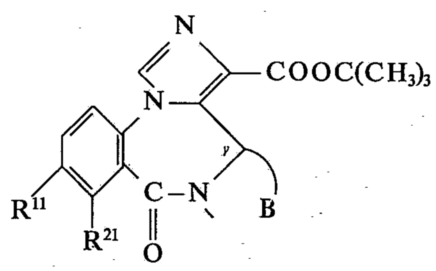

(Ia)

dans laquelle B a la signification indiquée ci-dessus, et ou bien $R^{11}$ représente un halogène et $R^{21}$ l'hydrogène, un groupe trifluorométhyle, amino, nitro, cyano ou alkyle inférieur, ou bien $R^{11}$ représente l'hydrogène, un groupe trifluorométhyle, amino, nitro, cyano ou alkyle inférieur et $R^{21}$ un halogène,
on remplace l'atome d'halogène par le groupe cyano ou bien encore, lorsque $R^{21}$ représente un halogène, par un groupe alkylthio inférieur, ou bien

d) dans un composé de formule générale

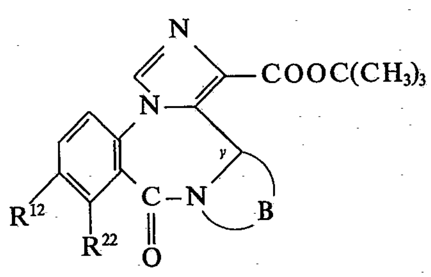

(Ib)

dans laquelle B a la signification indiquée ci-dessus, et ou bien $R^{12}$ représente un groupe amino et $R^{22}$ l'hydrogène, un halogène, un groupe trifluorométhyle, nitro, cyano ou alkyle inférieur, ou bien $R^{12}$ représente l'hydrogène, un halogène, un groupe trifluorométhyle, nitro, cyano ou alkyle inférieur et $R^{22}$ représente un groupe amino,
on remplace le groupe amino par un atome d'hydrogène ou d'halogène ou par un groupe cyano ou nitro, ou bien

e) on halogène un composé de formule générale

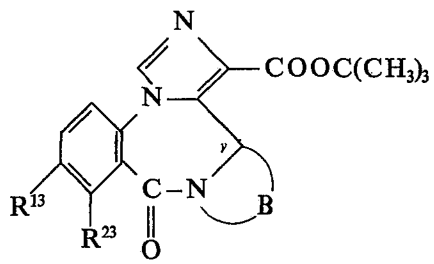

(Ic)

dans laquelle B a la signification indiquée ci-dessus, et l'un des symboles $R^{13}$ et $R^{23}$ représente un groupe amino et l'autre l'hydrogène,
en position $\alpha$ du groupe amino, ou bien

f) dans un composé de formule générale

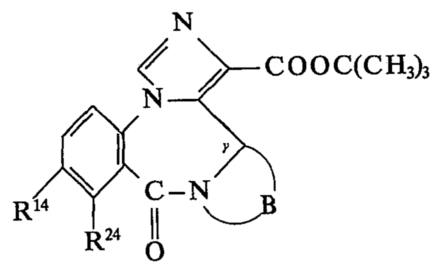

(Id)

dans laquelle $R^{14}$ représente l'hydrogène, un halogène, un groupe trifluorométhyle, nitro, cyano ou alkyle inférieur et $R^{24}$ représente un groupe alkylthio inférieur ou alkylsulfinyle inférieur, B ayant la signification indiquée ci-dessus,
on oxyde le groupe alkylthio inférieur en groupe alkylsulfinyle inférieur ou alkylsulfonyle inférieur ou le groupe alkylsulfinyle inférieur en groupe alkylsulfonyle inférieur et

g) si on le désire, on convertit un composé obtenu de formule générale I en un sel formé par addition avec un acide acceptable pour l'usage pharmaceutique.

2. Procédé selon la revendication 1, caractérisé en ce que l'on prépare des composés de formule I définie dans la revendication 1 dans laquelle A représente le groupe (a) défini dans la revendication 1, l'un des symboles $R^1$ et $R^2$ représente l'hydrogène et l'autre un groupe nitro ou cyano, et B représente un groupe diméthylène, triméthylène ou propénylèn, et l'atome de carbone marqué $\gamma$ présente la configuration (S) ou (R,S), par les procédés a), b) ou c) définis dans la revendication 1.

3. Procédé selon la revendication 1, caractérisé en ce que l'on prépare des composés de formule I définie dans la revendication 1 dans laquelle A représente le groupe (a) ou (d) définis dans la revendication 1, B représente un groupe diméthylène, $R^1$ représente l'hydrogène, un halogène ou un groupe trifluorométhyle et $R^2$ représente l'hydrogène, un halogène, un groupe trifluorométhyle ou alkyle inférieur, et l'atome de carbone marqué $\gamma$ présente la configuration (S) ou (R,S), et leurs sels formés par addition avec des acides acceptables pour l'usage pharmaceutique, par les procédés a), b) ou g) définis dans la revendication 1.

4. Procédé selon la revendication 1, caractérisé en ce que l'on prépare des composés de formule I définie dans la revendication 1 dans laquelle A représente le groupe (a) ou le groupe (d) définis dans la revendication 1, B représente un groupe triméthylène ou propénylène, $R^1$ représente l'hydrogène, un halogène ou un groupe trifluorométhyle et $R^2$ représente l'hydrogène, un halogène, un groupe trifluorométhyle ou alkyle inférieur, et l'atome de carbone marqué $\gamma$ présente la configuration (S) ou (R,S) par le pocédé a) défini dans la revendication 1.

5. Procédé selon la revendication 1, caractérisé en ce que l'on prépare des composés de formule I définie dans la revendication 1 dans laquelle A représente le groupe (b) ou (c) définis dans la revendication 1 et B représente un groupe diméthylène, triméthylène ou propénylène, et l'atome de carbone marqué $\gamma$ présente la configuration (S) ou (R,S), et leurs sels formés par addition avec des acides acceptables pour l'usage pharmaceutique, par les procédés a), b) ou g) définis dans la revendication 1.

6. Procédé selon la revendication 1, caractérisé en ce que l'on prépare des composés de formule I définie dans la revendication 1 dans laquelle A représente le groupe (a) défini dans la revendication 1, B représente un groupe diméthylène, triméthylène ou propénylèn, et ou bien $R^1$ représente un groupe alkyle inférieur et $R^2$ l'hydrogène, un halogène, un groupe trifluorométhyle, nitro, cyano ou alkyle inférieur, ou bien l'un des symboles $R^1$ et $R^2$ représente un groupe nitro ou cyano et l'autre un halogène, un groupe trifluorométhyle, nitro, cyano ou alkyle inférieur, et l'atome de carbone marqué $\gamma$

présente le configuration (S) ou (R,S), et leurs sels formés par addition avec des acides acceptables pour l'usage pharmaceutique, par les procédés a), b), c) ou g) définis dans la revendication 1.

7. Procédé selon la revendication 1, caractérisé en ce que l'on prépare des composés de formule I définie dans la revendication 1 dans laquelle A représente le groupe (a) ou (d) définis dans la revendication 1, B représente un groupe triméthylène ou propénylène, $R^1$ représente l'hydrogène, un halogène ou un groupe trifluorométhyle et $R^2$ représente l'hydrogène, un halogène, un groupe trifluorométhyle ou alkyle inférieur, et l'atome de carbone marqué $\gamma$ présente la configuration (S) ou la configuration (R,S) et leurs sels formés par addition avec des acides acceptables pour l'usage pharmaceutique, par les procédés b) ou g) définis dans la revendication 1.

8. Procédé selon la revendication 1, caractérisé en ce que l'on prépare des composés de formule I définie dans la revendication 1 dans laquelle A représente le groupe (a), (b), (c) ou (d) définis dans la revendication 1, $R^1$ représente l'hydrogène, un groupe amino ou un halogène et $R^2$ représente l'hydrogène, un halogène, un groupe trifluorométhyle, alkyle inférieur, cyano, nitro, amino, alkyloxy inférieur, alkylthio inférieur, alkylsulfinyle inférieur ou alkylsulfonyle inférieur.

9. Procédé selon la revendication 8, caractérisé en ce que l'on prépare des composés de formule I définie dans la revendication 1 dans laquelle A représente le groupe (a) défini dans la revendication 1, $R^1$ représente l'hydrogène ou un halogène et $R^2$ représente un halogène, un groupe trifluorométhyle ou alkyle inférieur.

10. Procédé selon l'une des revendications 1, 8 et 9, caractérisé en ce que l'on prépare des composés de formule I définie dans la revendication 1 dans laquelle B représente un groupe diméthylène ou triméthylène.

11. Procédé selon l'une des revendications 1 et 8 à 10, caractérisé en ce que l'on prépare un composé de formule I définie dans la revendication 1 dans laquelle l'atome de carbone marqué $\gamma$ présente la configuration (S).

12. Procédé selon la revendication 1, caractérisé en ce que l'on prépare le (S)-12,12a-dihydro-9-oxo-8-(trifluorométhyl)-9H,11H-azéto[2,1-c]imidazo[1,5-a][1,4]benzodiazépine-1-carboxylate de tert.-butyle.

13. Procédé selon la revendication 1, caractérisé en ce que l'on prépare le (S)-12,12a-dihydro-8-méthyl-9-oxo-9H,11H-azéto[2,1-c]imidazo[1,5-a][1,4]benzodiazépine-1-carboxylate de tert.-butyle.

14. Procédé selon la revendication 1, caractérisé en ce que l'on prépare le (S)-8-chloro-7-fluoro-11,12,13,13a-tétrahydro-9-oxo-9H-imidazo[1,5-a]pyrrolo[2,1-c][1,4]benzodiazépine-1-carboxylate de tert.-butyle.

15. Procédé selon la revendication 1, caractérisé en ce que l'on prépare le (S)-8-chloro-11,12,13,13a-tétrahydro-9-oxo-9H-imidazo[1,5-a]pyrrolo[2,1-c][1,4]benzodiazépine-1-carboxylate de tert.-butyle.

16. Procédé selon la revendication 1, caractérisé en ce que l'on prépare le (S)-8-éthyl-11,12,13,13a-tétrahydro-9-oxo-9H-imidazo[1,5-a]pyrrolo[2,1-c][1,4]benzodiazépine-1-carboxylate de tert.-butyle.

17. Procédé selon la revendication 1, caractérisé en ce que l'on prépare le (S)-11,12,13,13a-tétrahydro-9-oxo-8-(trifluorométhyl)-9H-imidazo[1,5-a]pyrrolo[2,1-c][1,4]benzodiazépine-1-carboxylate de tert.-butyle.

18. Procédé selon la revendication 1, caractérisé en ce que l'on prépare le (S)-8-bromo-11,12,13,13a-tétrahydro-9-oxo-9H-imidazo[1,5-a]pyrrolo[2,1-c][1,4]benzodiazépine-1-carboxylate de tert.-butyle.

19. Procédé selon la revendication 1, caractérisé en ce que l'on prépare le (S)-11,12,13,13a-tétrahydro-8-iodo-9-oxo-9H-imidazo[1,5-a]pyrrolo[2,1-c][1,4]benzodiazépine-1-carboxylate de tert.-butyle.

20. Procédé selon la revendication 1, caractérisé en ce que l'on prépare le (S)-8-chloro-12,12a-dihydro-9-oxo-9H,11H-azéto[2,1-c]imidazo[1,5-a][1,4]benzodiazépine-1-carboxylate de tert.-butyle.

21. Procédé selon la revendication 1, caractérisé en ce que l'on prépare le (S)-11,12,13,13a-tétrahydro-8-méthyl-9-oxo-9H-imidazo[1,5-a]pyrrolo[2,1-c][1,4]benzodiazépine-1-carboxylate de tert.-butyle.